# EUROPEAN PATENT APPLICATION

(11) **EP 4 733 314 A1**
(43) Date of publication of application: **29.04.2026**
(21) Application number: 24826320.4
(22) Date of filing: 21.06.2024
(51) Int. Cl.: C07K 14/605, A61P 3/00, A61K 38/00

(54) **NOVEL BISPECIFIC FUSION PROTEIN AND USE THEREOF**

(30) Priority: 22.06.2023 WO PCT/KR2023/008718
(71) Applicant: Slbigen Inc., Yeonsu-gu Incheon 21983 (KR)
(72) Inventor: SUNG, Young Chul, Seoul 04419 (KR); YANG, Sang In, Yongin-si, Gyeonggi-do 16823 (KR); ROH, Jaeil, Bucheon-si, Gyeonggi-do 14466 (KR)
(74) Representative: Latscha Schöllhorn Partner AG
(86) International application number: PCT/KR2024/008655
(87) International publication number: WO 2024/263005

(57) **Abstract**

The present invention relates to a novel bispecific fusion protein and a use thereof, and more particularly to a bispecific fusion protein comprising a GLP-1 analogue and a GLP-2 analogue, wherein the activities of GLP-1 and GLP-2 are appropriately regulated, thereby providing an improved effect on metabolic diseases such as obesity, diabetes, and metabolic dysfunction-associated steatohepatitis.

## Description

### TECHNICAL FIELD

The present invention relates to a novel bispecific fusion protein and its use, and more specifically, to a bispecific fusion protein comprising a GLP-1 analogue and a GLP-2 analogues, in which the activity of the GLP-1 analogue and the activity of the GLP-2 analogue are precisely regulated, and to the use thereof.

### BACKGROUND ART

GLP-1 and GLP-2 are peptide hormones generated from precursors of proglucagon through a series of tissue-specific proteolytic processes. Among these, GLP-1 is produced and secreted upon food intake in enteroendocrine L-cells of the small intestine and in specific neurons within the nucleus of the solitary tract of the brain stem. The primary product, GLP-1(1-37), is readily amidated and converted by cleavage into two truncated forms, GLP-1(7-36) amide and GLP-1(7-37), both having equivalent biological activity. Active GLP-1 plays a role in lowering blood glucose levels in a glucose-dependent manner and has therefore been developed and used as a therapeutic agent for type 2 diabetes. Similarly, GLP-2 is a peptide with a length of 33 amino acids, generated by special post-translational cleavage of proglucagon as in the case of GLP-1. It is produced in enteroendocrine L-cells of the small intestine and in various neurons of the central nervous system, and is secreted together with GLP-1 upon food intake. Administration of GLP-2 has been reported to promote intestinal growth and function, reduce bone resorption, and exert neuroprotective effects, and is currently being developed as a therapeutic agent for diseases such as short bowel syndrome, Crohn's disease, and osteoporosis.

As described above, GLP-1 was originally noted for its beneficial effects on glucose homeostasis in the treatment of type 2 diabetes (Gutniak et al., N. Engl. J. Med. 326: 1316-1322, 1992). However, similar to GLP-2, researches have found that GLP-1 reduces gastric secretion and motility, thereby suggesting its potential role in the treatment of short bowel syndrome (Kunkel et al., Neurogastroenterol. Motil. 23: 739-e328, 2011). Furthermore, GLP-1-induced activation of the GLP-1 receptor (GLP-1R) signaling pathway has been reported to promote intestinal growth and the fusion of crypt cells (Koehler et al., Cell Metabol. 21(3): 379-391, 2015). Based on these mechanisms, Naia Co. has been conducting a phase 1 clinical trial for short bowel syndrome using GLP-1-XTEN, in which a half-life extending peptide, XTEN, is conjugated to GLP-1. Moreover, it has been reported that combined administration of GLP-1 and GLP-2 exhibits synergistic effects on intestinal absorption compared to administration of each peptide alone (Madsen et al., Regul. Pept. 184: 30-39, 2013).

Meanwhile, metabolic dysfunction-associated steatotic liver disease (MASLD) is not a single disease but rather encompasses a spectrum of liver diseases ranging from simple steatosis without inflammation to chronic hepatitis and cirrhosis. MASLD is known to be closely related to obesity and the resulting insulin resistance. The disease ranges in severity from simple fatty liver without hepatocellular injury, to steatohepatitis with severe and persistent hepatocellular injury, to liver fibrosis or cirrhosis accompanied by ascites or jaundice. In advanced cases, progression to hepatocellular carcinoma has been reported. In addition, due to the widespread adoption of Western dietary habits, the incidence of this disease is rapidly increasing as a Western-type disorder. Although most MASLD cases are mild, approximately one out of four patients with severe fatty liver, if left untreated, gradually progresses to serious liver diseases such as cirrhosis or fibrosis. Therefore, it cannot be regarded as a trivial condition. The prevalence of MASLD varies depending on population characteristics, ranging from 10-24% in the general population to 58-74% in obese individuals. However, as of now, there are no approved drugs for the treatment of MASLD or metabolic dysfunction-associated steatohepatitis, and thus the development of therapeutic agents for MASLD is considered an urgent and unmet need.

In this regard, the present inventors have previously developed a bispecific protein simultaneously targeting GLP-1R and GLP-2R (Korean Patent No. 10-2349718).

### DETAILED DESCRIPTION OF THE INVENTION

### TECHNICAL PROBLEM

However, although the bispecific fusion protein can be used broadly for various metabolic diseases such as short bowel syndrome, obesity, type 2 diabetes, and metabolic dysfunction-associated steatohepatitis, no disclosure has been made regarding a bispecific fusion protein in a form more suitable for the treatment of metabolic diseases.

Accordingly, the present invention has been devised to solve the above-mentioned and other problems, and it is an object of the invention to provide a novel bispecific fusion protein which maintains the functions of GLP-1 and GLP-2 while having an activity more suitable for the treatment of metabolic diseases. However, the scope of protection of the present invention is not limited to this objective.

### TECHNICAL SOLUTION

According to one aspect of the present invention, there is provided a bispecific fusion protein comprising a GLP-1 analogue and a GLP-2 analogues, wherein the activity of the GLP-1 analogue is 40% or more compared to wild-type GLP-1 (human GLP-1 peptide), and the activity of the GLP-2 analogue is 50% or less compared to wild-type GLP-2 (human GLP-2 peptide).

According to another aspect of the present invention, there is provided a bispecific fusion protein generated by dimerization of a first fusion protein comprising a GLP-1 analogue linked to an antibody Fc region and a second fusion protein comprising a GLP-2 analogue linked to an antibody Fc region, wherein the activity of the GLP-1 analogue is 40% or more compared to wild-type GLP-1 (human GLP-1 peptide), and the activity of the GLP-2 analogue is 50% or less compared to wild-type GLP-2 (human GLP-2 peptide).

According to one aspect of the present invention, there is provided a composition comprising the bispecific fusion protein.

According to another aspect of the present invention, there is provided a pharmaceutical composition for treating metabolic diseases, comprising one or more of the bispecific fusion proteins as an active ingredient.

According to another aspect of the present invention, there is provided one of the bispecific fusion proteins for use in the treatment of metabolic diseases.

According to another aspect of the present invention, there is provided the use of one of the bispecific fusion proteins in the manufacture of a therapeutic agent for metabolic diseases.

According to another aspect of the present invention, there is provided a method for treating a metabolic disease in a subject suffering from a metabolic disease, comprising administering a therapeutically effective amount of the bispecific fusion protein to the subject.

### EFFECT OF THE INVENTION

The bispecific fusion protein of the present invention can be used very effectively for the treatment of metabolic diseases such as metabolic syndrome, obesity, type 1 diabetes, type 2 diabetes, metabolic dysfunction-associated steatotic liver disease, and liver fibrosis when administered *in vivo.*

### BRIEF DESCRIPTION OF DRAWINGS

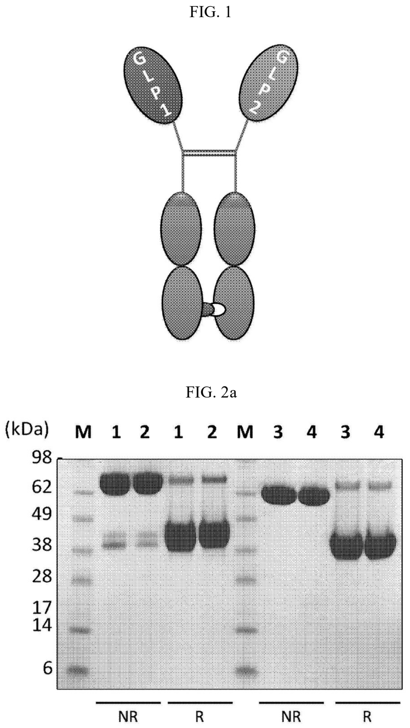

FIG. 1a is a schematic diagram showing the general structure of a bispecific fusion protein according to one embodiment of the present invention.
FIG. 2a shows a series of gel images obtained from SDS-PAGE analysis under non-reducing (NR) and reducing (R) conditions after purification of GLP-2-Fc homodimer (left, comparative example) and MG12-5 (right, including a knobs-into-holes structure) of the present invention:
   M: size marker;
   1 & 2: GLP-2-Fc homo (10 µg); and
   3 & 4: MG12-5 (10 µg).

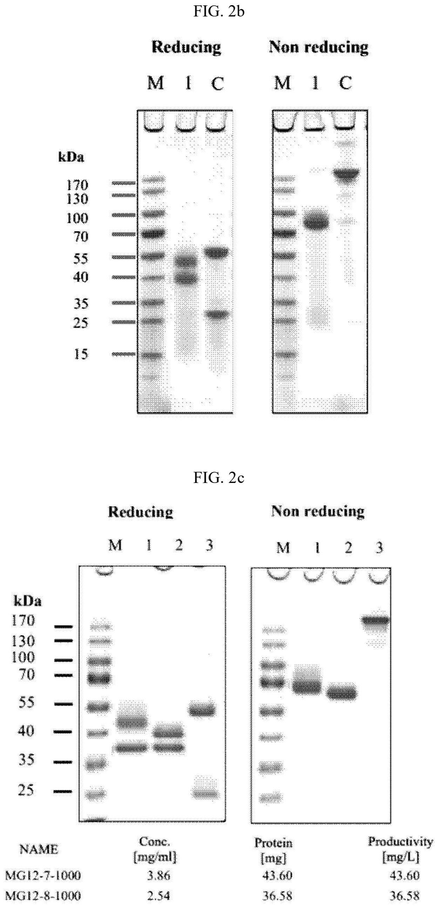

FIG. 2b shows a series of gel images obtained from SDS-PAGE analysis under reducing and non-reducing conditions of MG12-6 according to one embodiment of the present invention:
   M: size marker;
   1: MG12-6; and
   C: Ctrl (3 µg) - Herceptin.
FIG. 2c shows a series of gel images obtained from SDS-PAGE analysis under reducing and non-reducing conditions of MG12-7 and MG12-8 according to one embodiment of the present invention:
   M: size marker;
   1: MG12-7;
   2: MG12-8; and
   3: Ctrl (3 µg) - Herceptin.

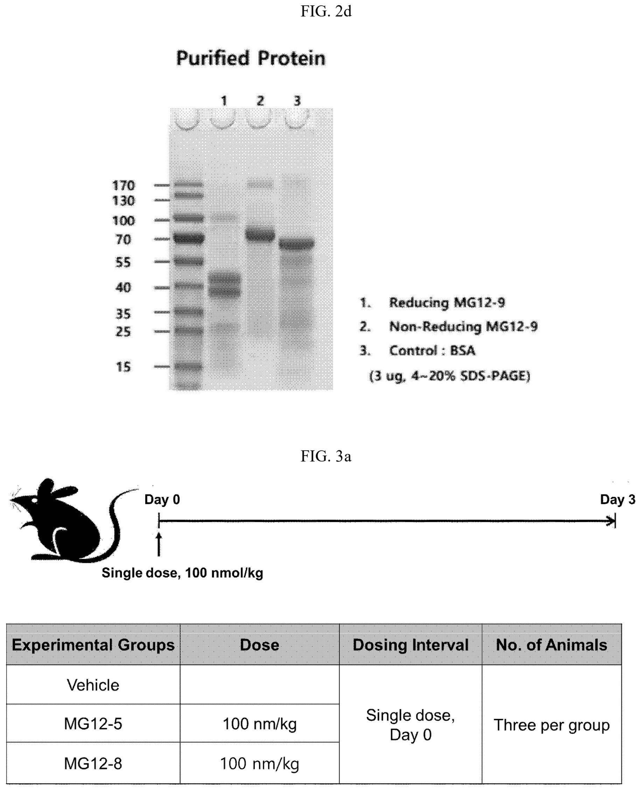

FIG. 2d shows a gel image obtained from SDS-PAGE analysis under reducing and non-reducing conditions of MG12-9 according to one embodiment of the present invention:
   1: Reducing MG12-9;
   2: Non-reducing MG12-9; and
   3: Ctrl (3 µg) - BSA.

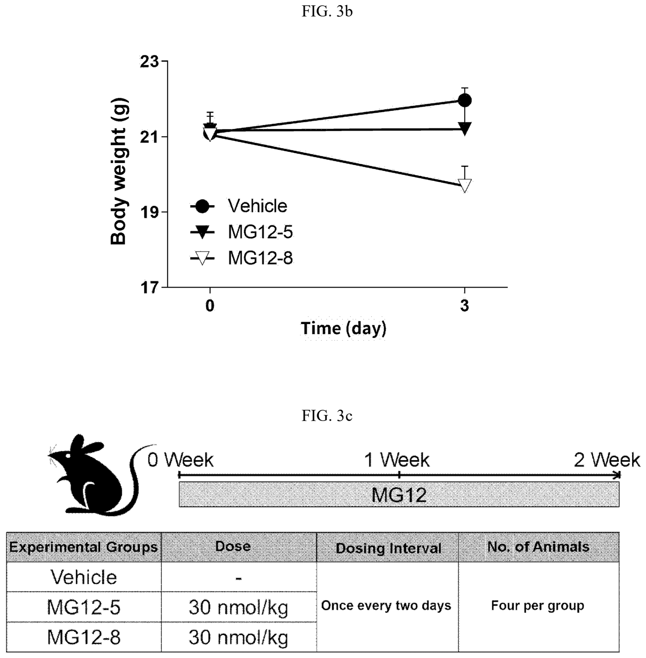

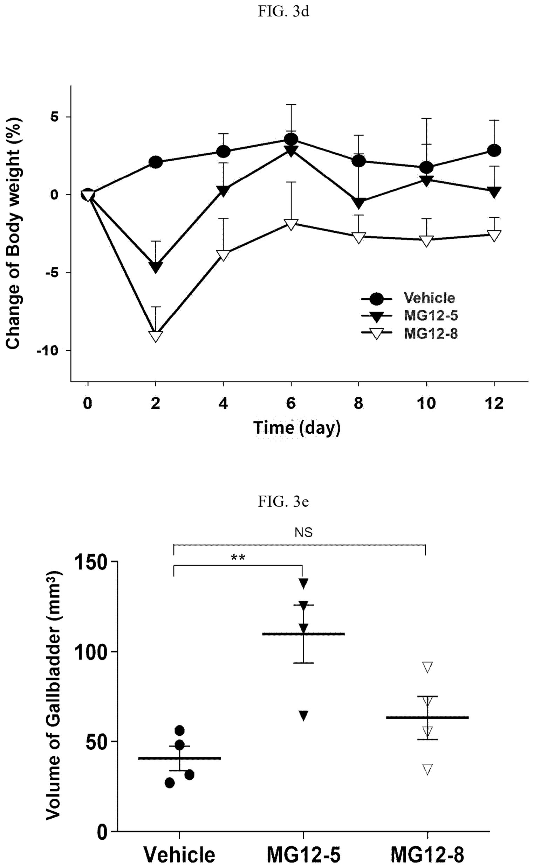

FIG. 3a is a schematic diagram summarizing the experimental schedule, drug dosage, and dosing interval for analyzing the weight loss effect of a single administration of the bispecific fusion protein (MG12-8) according to one embodiment of the present invention. FIG. 3b is a graph showing changes in body weight of experimental animals obtained according to the schedule in FIG. 3a. FIG. 3c is a schematic diagram summarizing the experimental schedule, drug dosage, and dosing interval for analyzing the weight loss effect of repeated administration of the bispecific fusion protein (MG12-8) according to one embodiment of the present invention. FIG. 3d is a graph showing changes in body weight of experimental animals obtained according to the schedule of FIG. 3c. FIG. 3e is a graph showing gallbladder volume measured after sacrifice of the experimental animals following the experiment of FIG. 3d. "NS" indicates not statistically significant; ** indicates statistical significance at *p* < 0.01; and error bars represent standard error of the mean (SEM).

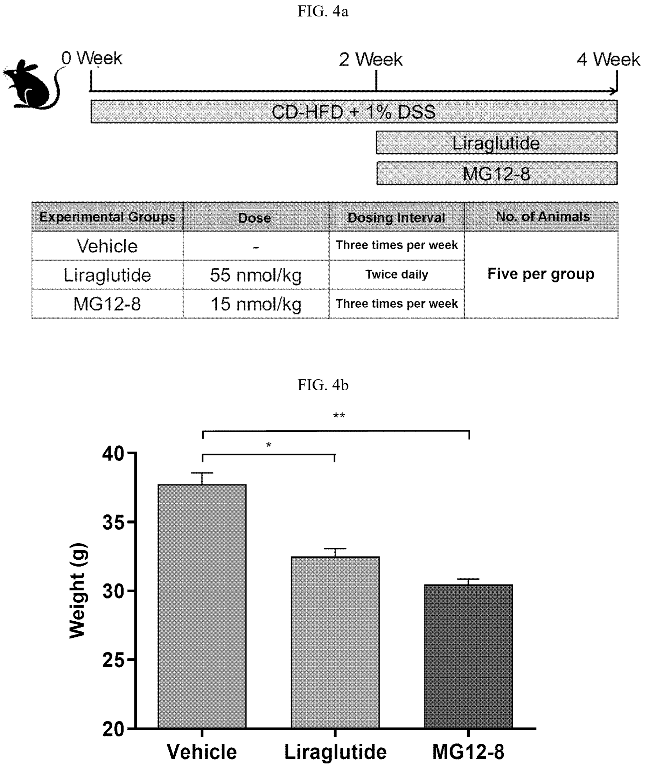

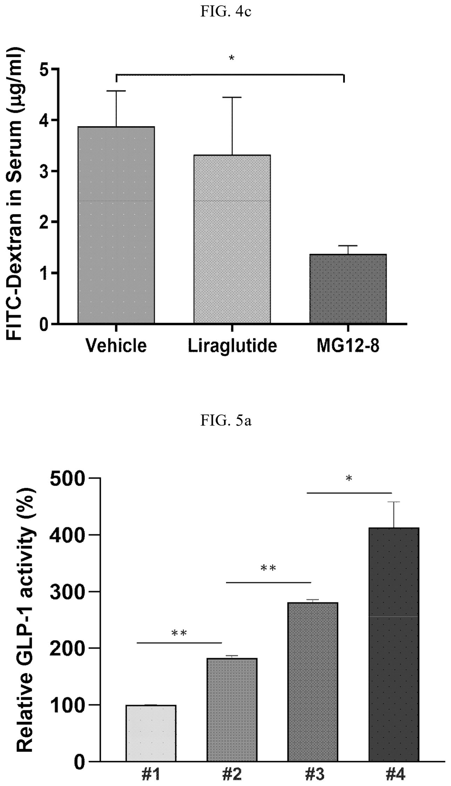

FIG. 4a is a schematic diagram summarizing the experimental schedule, drug dosage, and dosing interval for evaluating the therapeutic effect of the bispecific fusion protein of the present invention on MASLD accompanied by intestinal leakage. FIG. 4b is a graph showing body weight of experimental animals measured after the animal experiment according to the schedule of FIG. 4a. FIG. 4c is a graph showing the serum fluorescence intensity of FITC-Dextran, orally administered 4 hours before sacrifice of the experimental animal of FIG. 4b..

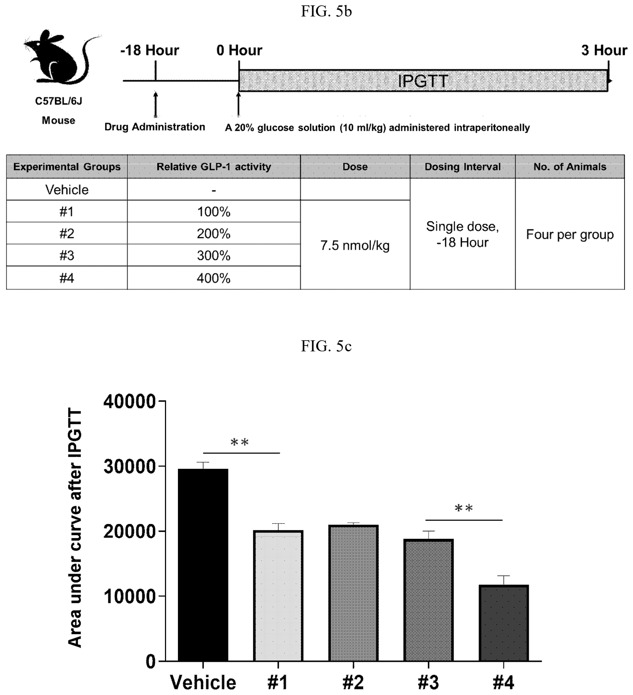

FIG. 5a is a graph showing the *in vitro* GLP-1 activity of various bispecific fusion protein samples prepared according to one embodiment of the present invention. FIG. 5b is a schematic diagram summarizing the experimental schedule, drug dosage, and dosing interval for intraperitoneal glucose tolerance test (IPGTT) *in vivo* to analyze GLP-1 activity of various bispecific fusion proteins with different *in vitro* GLP-1 activities. FIG. 5c is a graph showing the results of the IPGTT conducted according to the schedule of FIG. 5b. In the graphs, * indicates statistical significance at *p* < 0.05; ** indicates statistical significance at *p* < 0.01; and error bars represent standard error of the mean (SEM).

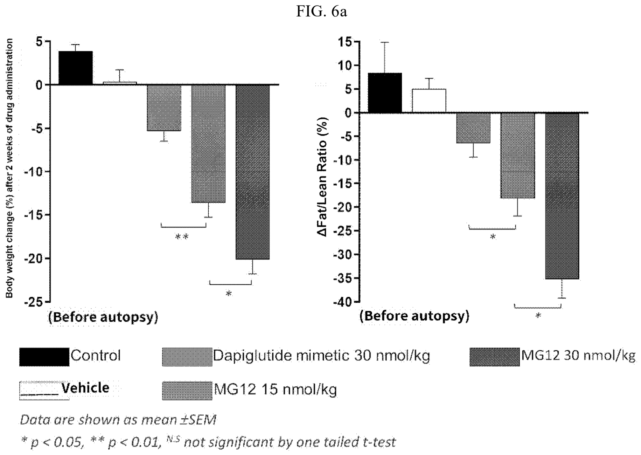

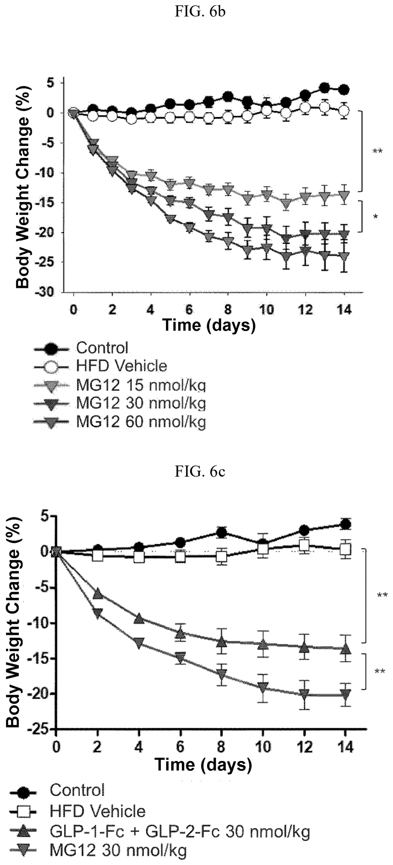

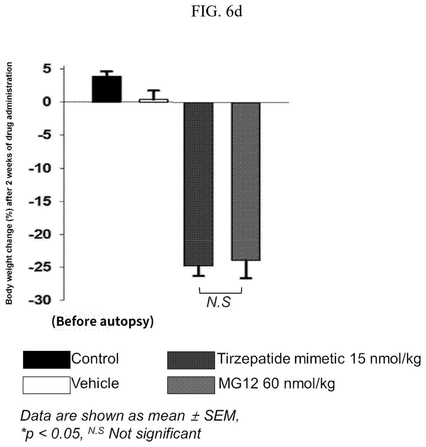

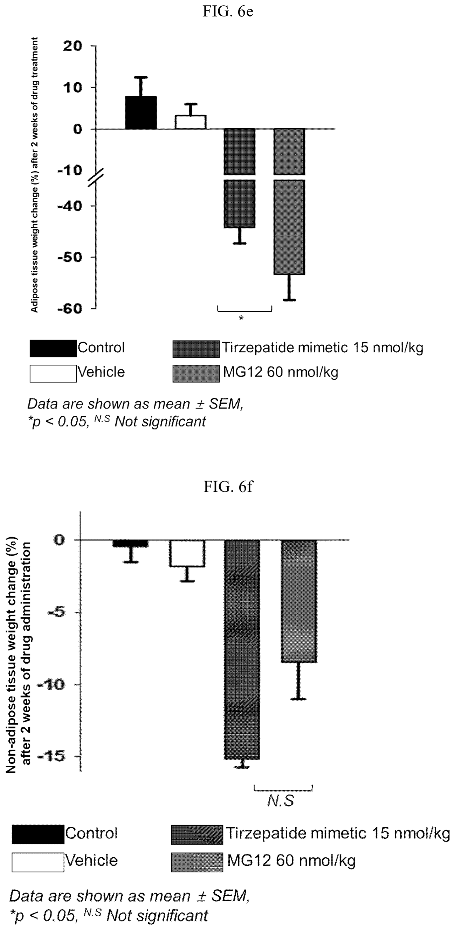

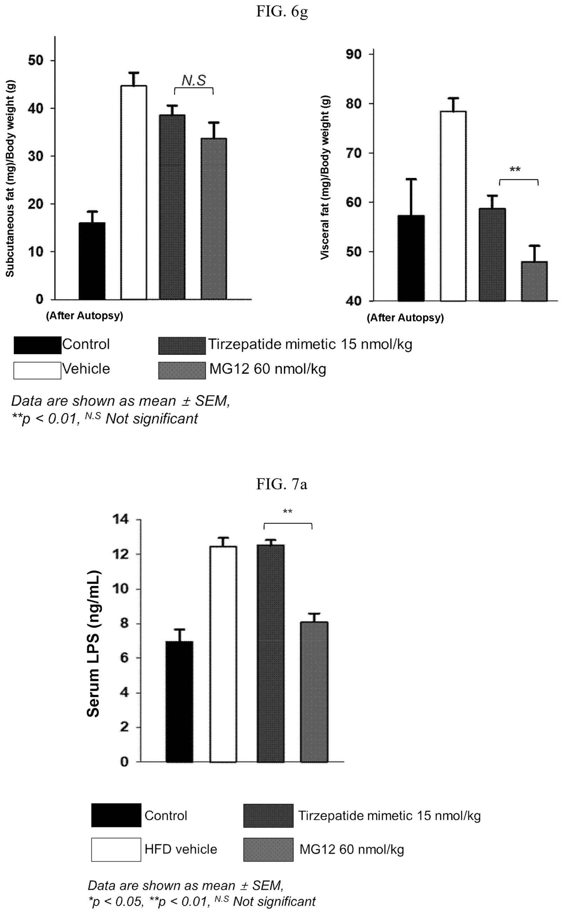

FIG. 6a shows a series of graphs showing the changes in body weight (left) and the fat-to-lean mass ratio (right) following administration of the bispecific fusion protein of the present invention, compared to a known GLP-1R/GLP-2R dual agonist, Dapiglutide mimetic. FIG. 6b is a graph showing changes in body weight according to the dosage of the bispecific fusion protein of the present invention. FIG. 6c is a graph showing the effect of the bispecific fusion protein of the present invention on body weight changes compared to simple co-administration of GLP-1-Fc and GLP-2-Fc at equivalent doses. FIG. 6d is a graph showing body weight changes in diet-induced obese (DIO) model animals after administration of the bispecific fusion protein of the present invention, compared to the GLP-1R/GIPR dual agonist, Tirzepatide mimetic. FIG. 6e is a graph showing changes in fat tissue weight of the animals in FIG. 6d. FIG. 6f is a graph showing changes in lean tissue weight of the animals in FIG. 6d. FIG. 6g is a graph showing the subcutaneous fat ratio (left) and visceral fat ratio (right) of diet-induced obese model animals after administration of the bispecific fusion protein of the present invention. Tirzepatide mimetic was used as a positive control. In the graphs, N.S indicates not statistically significant; * indicates *p <* 0.05; and ** indicates *p* < 0.01.

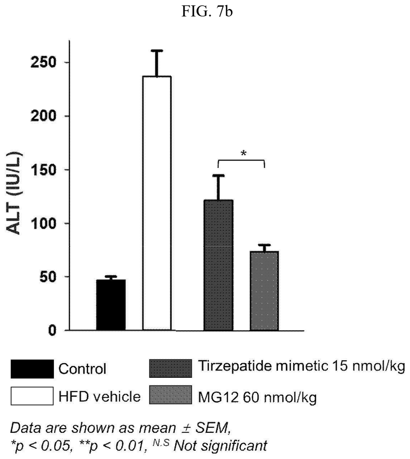

FIG. 7a is a graph showing the serum LPS concentration measured to analyze the endotoxemia-reducing effect of the bispecific fusion protein of the present invention in diet-induced obese model animals. FIG. 7b is a graph showing serum ALT concentration measured to analyze the hepatoprotective effect in the experimental animals of FIG. 7a. In the graphs, * indicates *p* < 0.05 and ** indicates *p* < 0.01.

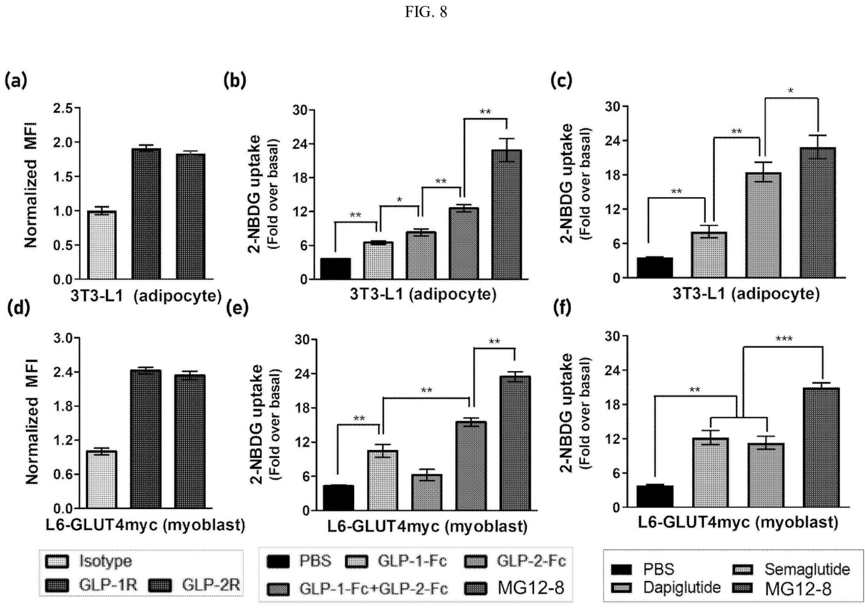

FIG. 8 is a series of graphs showing the results of insulin-stimulated glucose uptake analysis of the bispecific fusion protein of the present invention: (a) normalized mean fluorescence intensity of surface expression of GLP-1R and GLP-2R in differentiated 3T3-L1 adipocytes; (b, c) insulin-stimulated glucose uptake in differentiated 3T3-L1 adipocytes; (d) normalized mean fluorescence intensity of surface expression of GLP-1R and GLP-2R in differentiated L6-GLUT4myc myoblasts; and (e, f) insulin-stimulated glucose uptake in differentiated L6-GLUT4myc myoblasts. In the graphs, * indicates *p* < 0.05 and ** indicates *p* < 0.01.

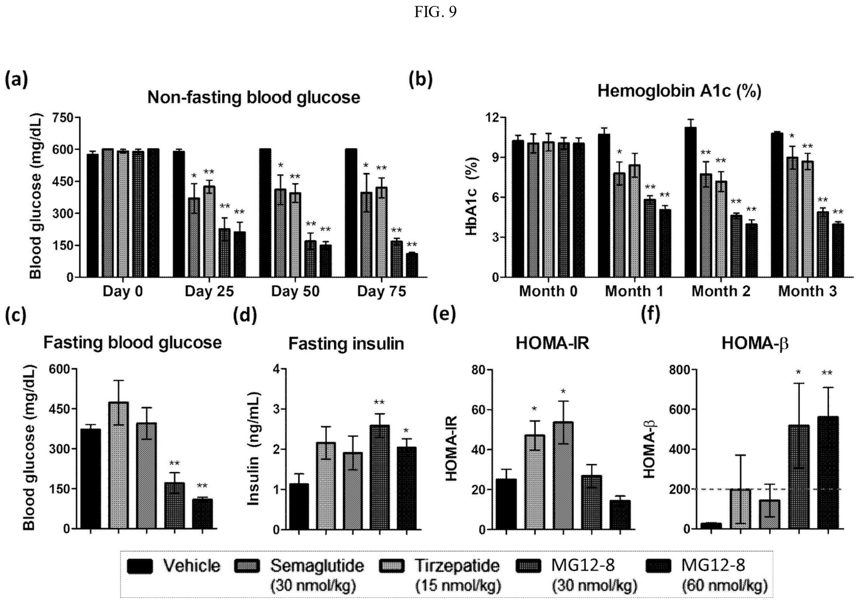

FIG. 9 is a series of graphs showing the effects of administration of the bispecific fusion protein of the present invention on blood glucose, glycated hemoglobin, and insulin resistance: (a) non-fasting blood glucose levels, (b) glycated hemoglobin (HbA1c) levels, (c) fasting blood glucose levels, (d) fasting insulin concentration, (e) HOMA-IR score, and (f) HOMA-β score. In the graphs, * indicates *p* < 0.05 and ** indicates *p* < 0.01.

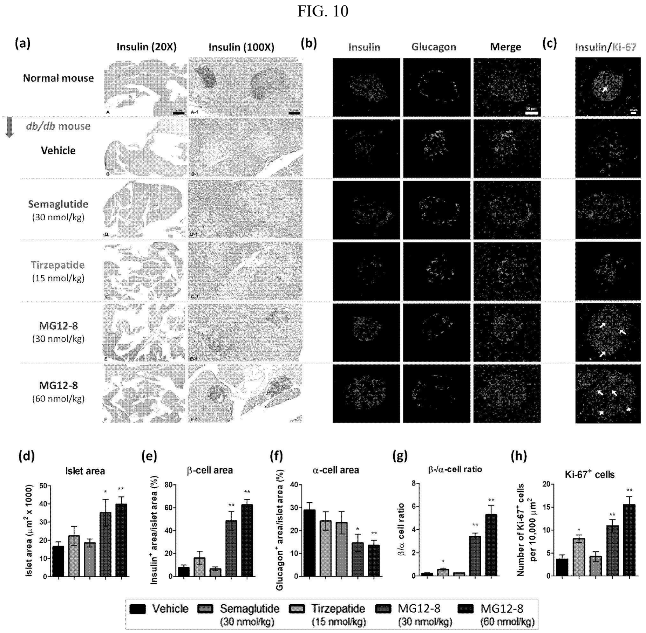

FIG. 10 shows histological analysis results of the pancreas excised after the experiment of FIG. 9: (a-c) immunohistochemical staining results of pancreatic tissue sections for (a) insulin, (b) insulin and glucagon, and (c) insulin and Ki-67 positive cells; (d) islet area, (e) β-cell area, (f) α-cell area, (g) β-/α-cell ratio, and (h) the number of Ki-67 positive cells per 10,000 µm². In the graphs, * indicates *p* < 0.05 and ** indicates *p* < 0.01.

### BEST MODE FOR CARRYING OUT THE INVENTION

### Definitions of Terms:

The term "GLP-1" as used herein is an abbreviation for glucagon-like peptide-1, a peptide hormone of 30 or 31 amino acids in length that is derived from tissue-specific post-translational processing of proglucagon peptides. GLP-1 is produced and secreted in enteroendocrine L-cells of the small intestine and in certain neurons within the nucleus of the solitary tract of the brain stem upon food intake. The initial product, GLP-1(1-37), is readily amidated and converted by cleavage into two truncated forms, GLP-1(7-36) amide and GLP-1(7-37), both having equivalent biological activity. Active GLP-1 contains two α-helical regions at amino acid positions 13-20 and 24-35, connected by a linker region. Since GLP-1 functions to lower blood glucose levels in a glucose-dependent manner, it has been developed and used as a therapeutic agent for type 2 diabetes. However, *in vivo,* GLP-1 is rapidly degraded by dipeptidyl peptidase-4 (DPP-4), resulting in a half-life of only 2 minutes, and thus its effectiveness as a native peptide is highly limited.

The term "GLP-2" as used herein refers to a 33-amino acid peptide generated by post-translational cleavage of proglucagon and produced in enteroendocrine L-cells of the small intestine and various neurons of the central nervous system, like GLP-1. GLP-2 is secreted together with GLP-1 upon food intake. GLP-2 has been reported to enhance intestinal growth and function, reduce bone resorption, and exert neuroprotective effects when administered, and is currently under development as a therapeutic agent for diseases such as short bowel syndrome, Crohn's disease, and osteoporosis.

The term "GLP-1 analogue" as used herein refers to a protein that biologically performs the function of GLP-1, meaning a protein capable of binding to a GLP-1/Exendin-4 receptor and mediating downstream signaling. It is also referred to as a "GLP-1 receptor agonist."

The term "GLP-2 analogue" as used herein refers to a protein that biologically performs the function of GLP-2, meaning a protein capable of binding to a GLP-2 receptor and mediating downstream signaling. It is also referred to as a "GLP-2 receptor agonist."

The term "fusion protein" as used in the present invention means a recombinant protein in which two or more proteins, or domains responsible for specific functions within the proteins, are linked so that each protein or domain performs its inherent function.

The term "half-life extending moiety" as used herein refers to a functional group conjugated to a recombinant protein for improving the *in vivo* half-life of the recombinant protein. Examples of such moieties include antibody Fc region (Capon et al., Nature 337: 525-531, 1989), PEG (Caliceti and Veronese, *Adv. Drug Delivery Rev.* 55: 1261-1277, 2003), XTEN (Schellenberger et al., Nat. Biotechnol. 27: 1186-1190, 2009), PAS (Pro-Ala-Ser, Schlapschy et al., Protein Eng. Des. Sel. 26: 489-501, 2013), ELP (elastin-like peptide, Floss et al., Trends Biotechnol. 28: 37-45, 2010), glycine-rich HAP (homo-amino-acid polymer, Schlapschy et al., Protein Eng. Des. Sel. 20: 273-284, 2007), GLK (gelatine-like protein, Huang et al., Eur. J. Pharm. Biopharm. 74(3): 435-441, 2010), and serum albumin (Sheffield et al., Cell Physiol. Biochem. 45(2): 772-782, 2018). Such moieties have been well described in review articles (Strohl, W. R., BioDrugs 29(4): 215-239, 2015). Articles and reviews about each of the above element are incorporated into this document by reference.

The term "antibody Fc region" refers to the crystallizable fragment generated when an antibody is cleaved with papain. It interacts with cell surface receptors known as Fc receptors and with certain proteins of the complement system. The Fc region is a homodimeric structure consisting of the CH2 and CH3 domains of the heavy chain, connected at the hinge region by interchain disulfide bonds. The Fc region of IgG contains multiple N-glycan attachment sites, which are known to play an important role in Fc receptor-mediated functions.

The term "hybrid Fc region" refers to an Fc region peptide generated by combining parts of different Ig Fc region subtypes, and may differ from wild-type Fc regions in binding ability to Fc receptors and complement.

The term "Exendin" refers to a 39-amino acid peptide isolated from the venom of the lizard Heloderma suspectum. Exendin-4 shares 50% sequence identity with GLP-1 and, as a member of the glucagon peptide family, acts as a GLP-1 receptor agonist with equivalent functionality to GLP-1. Exendin-4 is also known as "exenatide." Exendin-3 is a variant of Exendin-4 in which the second and third amino acids are substituted with serine and aspartic acid, respectively.

The term "Lixisenatide" refers to one of the GLP-1 receptor agonists, manufactured by Sanofi and marketed as Lyxumia^{®} in Europe and Adlyxin^{®} in the United States, as a once-daily injectable drug for the treatment of type 2 diabetes.

The term "Albiglutide" refers to one of the GLP-1 receptor agonists, marketed by GSK as Eperzan^{®} in Europe and Tanzeum^{®} in the United States for the treatment of type 2 diabetes.

The term "Liraglutide" refers to a GLP-1 receptor agonist marketed by Novo Nordisk as Victoza^{®} for the treatment of type 2 diabetes and obesity.

The term "Taspoglutide" refers to a GLP-1 receptor agonist co-developed by Ipsen and Roche as a therapeutic for type 2 diabetes. It is a GLP-1(7-36) derivative in which the 8th and 35th alanine residues are methylated and the terminal amino acid is amidated. When manufactured as a fusion protein with another peptide, the C-terminus may be a standard carboxyl group rather than amidated.

The term "Semaglutide" refers to a GLP-1R agonist used as an oral hypoglycemic agent for type 2 diabetes as well as an anti-obesity drug for long-term weight management. It was developed by Novo Nordisk in 2012 and approved for sale in the United States in 2017.

The term "Tirzepatide" refers to a type of GLP-1R agonist used for type 2 diabetes and weight loss, developed by Eli Lilly and approved in the United States in 2022.

The term "Dapiglutide" refers to a GLP-1R/GLP-2R dual agonist developed by Zealand Pharma, currently undergoing phase 1 clinical trials.

The term "XTEN" refers to an unstructured, low-immunogenicity peptide comprising repeats of six amino acids, developed by Amunix to extend the *in vivo* half-life of protein drugs. Typically consisting of 144 amino acids or multiples thereof, XTEN is described in US2010/0239554A1.

The term "Teduglutide" refers to a GLP-2 analogue in which the second amino acid alanine (A) of GLP-2 is substituted with glycine (G). It is marketed as Gattex^{®} in the United States and Revestive^{®} in Europe as a therapeutic agent for short bowel syndrome.

The term "Glepaglutide" refers to a GLP-2 analogue with an extended half-life, developed for the treatment of short bowel syndrome and currently in phase 3 clinical trials.

The term "GLP-2 analogue 10" refers to a GLP-2 analogue in which the 11^{th} and 18^{th} amino acids are substituted with cysteines, forming a stabilized structure through intramolecular crosslinking of the cross-linkers in the lapidated molecule through thiol groups in the two introduced cysteines, and further comprising the C-terminal nine amino acids of Exendin-4 at the C-terminus (Yang et al., J. Med. Chem. 61: 3218-3223, 2018).

The term "linker peptide" refers to an unstructured peptide used to connect two or more proteins or peptides having different biological activities for the construction of a fusion protein.

The term "metabolic disorder" as used herein is a collective term for diseases caused by abnormalities in metabolic processes by which energy is obtained from food *in vivo,* particularly including disorders arising from impaired regulation of carbohydrate metabolism such as obesity and diabetes. Causes of such metabolic disorders include genetic and environmental factors, with carbohydrate-rich diets recently being identified as a primary cause in addition to genetic predisposition. Examples of metabolic disorders include type 1 diabetes, type 2 diabetes, obesity, metabolic syndrome, metabolic dysfunction-associated steatotic liver disease, metabolic dysfunction-associated steatohepatitis, and cirrhosis.

The term "metabolic syndrome" as used herein refers to a condition presumed to b e caused by insulin resistance, characterized by abnormalities in two or more of cholesterol, b lood pressure, and blood glucose. It conceptualizes the clustering of risk factors for cardiovas cular disease and type 2 diabetes into a single disease entity. As a useful concept encompassi ng insulin resistance (IR), associated metabolic abnormalities, and diverse clinical phenotype s, metabolic syndrome is known to increase the risk of atherosclerosis, myocardial infarction, stroke, and type 2 diabetes if left untreated.

### Detailed descriptions of the invention:

According to one aspect of the present invention, there is provided a bispecific fusion protein comprising a GLP-1 analogue and a GLP-2 analogue, wherein the activity of the GLP-1 analogue is at least 40% compared to wild-type GLP-1 (human GLP-1 peptide), and the activity of the GLP-2 analogue is 50% or less compared to wild-type GLP-2 (human GLP-2 peptide).

According to another aspect of the present invention, there is provided a bispecific fusion protein generated by dimerization of a first fusion protein comprising a GLP-1 analogue linked to an antibody Fc region and a second fusion protein comprising a GLP-2 analogue linked to an antibody Fc region, wherein the activity of the GLP-1 analogue is at least 40% compared to wild-type GLP-1 (human GLP-1 peptide), and the activity of the GLP-2 analogue is 50% or less compared to wild-type GLP-2 (human GLP-2 peptide).

In the bispecific fusion protein, the activity of the GLP-1 analogue is preferably at least 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% compared to wild-type GLP-1 under *in vitro* conditions, and may also have an activity of more than 100% and up to 400%.

More specifically, the activity of the GLP-1 analogue under *in vitro* conditions may be 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, or 120% compared to wild-type GLP-1, and may be defined by a certain range having any of the above percentages as a lower limit and a greater percentage as an upper limit.

Furthermore, the activity of the GLP-1 analogue may optionally exceed 120% under *in vitro* conditions, and more specifically may be 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%, 151%, 152%, 153%, 154%, 155%, 156%, 157%, 158%, 159%, 160%, 161%, 162%, 163%, 164%, 165%, 166%, 167%, 168%, 169%, 170%, 171%, 172%, 173%, 174%, 175%, 176%, 177%, 178%, 179%, 180%, 181%, 182%, 183%, 184%, 185%, 186%, 187%, 188%, 189%, 190%, 191%, 192%, 193%, 194%, 195%, 196%, 197%, 198%, 199%, 200%, 201%, 202%, 203%, 204%, 205%, 206%, 207%, 208%, 209%, 210%, 211%, 212%, 213%, 214%, 215%, 216%, 217%, 218%, 219%, 220%, 221%, 222%, 223%, 224%, 225%, 226%, 227%, 228%, 229%, 230%, 231%, 232%, 233%, 234%, 235%, 236%, 237%, 238%, 239%, 240%, 241%, 242%, 243%, 244%, 245%, 246%, 247%, 248%, 249%, 250%, 251%, 252%, 253%, 254%, 255%, 256%, 257%, 258%, 259%, 260%, 261%, 262%, 263%, 264%, 265%, 266%, 267%, 268%, 269%, 270%, 271%, 272%, 273%, 274%, 275%, 276%, 277%, 278%, 279%, 280%, 281%, 282%, 283%, 284%, 285%, 286%, 287%, 288%, 289%, 290%, 291%, 292%, 293%, 294%, 295%, 296%, 297%, 298%, 299%, 300%, 301%, 302%, 303%, 304%, 305%, 306%, 307%, 308%, 309%, 310%, 311%, 312%, 313%, 314%, 315%, 316%, 317%, 318%, 319%, 320%, 321%, 322%, 323%, 324%, 325%, 326%, 327%, 328%, 329%, 330%, 331%, 332%, 333%, 334%, 335%, 336%, 337%, 338%, 339%, 340%, 341%, 342%, 343%, 344%, 345%, 346%, 347%, 348%, 349%, 350%, 351%, 352%, 353%, 354%, 355%, 356%, 357%, 358%, 359%, 360%, 361%, 362%, 363%, 364%, 365%, 366%, 367%, 368%, 369%, 370%, 371%, 372%, 373%, 374%, 375%, 376%, 377%, 378%, 379%, 380%, 381%, 382%, 383%, 384%, 385%, 386%, 387%, 388%, 389%, 390%, 391%, 392%, 393%, 394%, 395%, 396%, 397%, 398%, 399%, or 400%, and may exceed 400% as well. The *in vitro* GLP-1 activity of the bispecific fusion protein according to an embodiment of the present invention may also be defined by a certain range having any of the above percentages as a lower limit and a greater percentage as an upper limit.

In the bispecific fusion protein, the *in vitro* activity of the GLP-2 analogue may be at least 2% compared to wild-type GLP-2.

In the bispecific fusion protein, the activity of the GLP-2 analogue may be 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50% or less, compared to wild-type GLP-2, and may have a defined range with any of the above percentages as the lower limit and a greater percentage as the upper limit.

The bispecific fusion protein may include a half-life extending moiety, which may be inserted between the GLP-1 analogue and the GLP-2 analogue, or attached to the N-terminus or C-terminus of the entire fusion protein. Preferably, the half-life extending moiety may be an antibody Fc region, PEG, XTEN, PAS (Pro-Ala-Ser), ELP (elastin-like peptide), glycine-rich HAP (homo-amino-acid polymer), GLP (gelatine-like protein), or serum albumin.

The bispecific fusion protein may not be a proglucagon or its analogue in which GLP-1 and GLP-2 naturally expressed *in vivo* are linked via an intermediate peptide, but rather a fusion protein in which a GLP-1 analogue and a GLP-2 analogue are directly linked or linked by a linker peptide other than the intermediate peptide.

In the bispecific fusion protein, the GLP-1 analogue may be GLP-1, Exendin-3, Exendin-4, a GLP-1/Exendin-4 hybrid peptide, GLP-1-XTEN, Exendin-4-XTEN, Lixisenatide, Albiglutide, Liraglutide, or Taspoglutide, or a continuous repeat thereof in which one or more of these are connected. Such continuous repeats of GLP-1 analogues may enhance GLP-1 activity or increase length, thereby enabling quality control related to the production of bispecific proteins (making it easier to distinguish whether the produced protein is a heterodimer or homodimer), and also simplifying the manufacturing process compared to conventional half-life extension technologies such as PEGylation, thereby reducing production costs. The number of repeating units in such continuous repeats of GLP-1 analogues may be adjusted according to the required GLP-1 activity.

In the bispecific fusion protein, the GLP-1 analogue may comprise the amino acid sequence set forth in SEQ ID NO: 1 or 2.

In the bispecific fusion protein, the Exendin-3 may comprise the amino acid sequence set forth in SEQ ID NO: 3.

In the bispecific fusion protein, the Exendin-4 may comprise the amino acid sequence set forth in SEQ ID NO: 4.

In the bispecific fusion protein, the GLP-1/Exendin-4 hybrid may comprise the amino acid sequence set forth in SEQ ID NO: 5.

In the bispecific fusion protein, the Lixisenatide may comprise the amino acid sequence set forth in SEQ ID NO: 6.

In the bispecific fusion protein, the Exendin-4-XTEN may comprise the amino acid sequence set forth in SEQ ID NO: 7.

In the bispecific fusion protein, the Albiglutide may comprise the amino acid sequence set forth in SEQ ID NO: 8.

In the bispecific fusion protein, the Liraglutide may comprise the amino acid sequence set forth in SEQ ID NO: 9.

In the bispecific fusion protein, the Taspoglutide may comprise the amino acid sequence set forth in SEQ ID NO: 10.

In the bispecific fusion protein, the GLP-1 continuous repeat may comprise the amino acid sequence set forth in SEQ ID NO: 11.

In the bispecific fusion protein, the antibody Fc region may be an antibody Fc region with a heterodimerization moiety, and the dimerization moiety may be Knobs-into-Holes (KiH), KiHS-S, HA-TF, ZW1, 7.8.60, DD-KK, EW-RVT, EW-RVTS-S, SEED, or A107.

The term "Knobs-into-Holes (KiH)" as used herein refers to a design strategy in antibody engineering for heterodimerization of heavy chains, employed in the production of recombinant proteins or IgG antibodies including Fc regions. In the KiH technology, when the two fusion proteins forming a heterodimer are distinguished as a first fusion protein and a second fusion protein: The first fusion protein may be a first modified Fc region in which the 22^{nd} amino acid threonine (T) in the CH3 domain is substituted with tryptophan (W) (T366W, Knob), and the second fusion protein may be a second modified Fc region in which the 22^{nd} amino acid threonine (T) in the CH3 domain is substituted with serine (S) (T366S), the 24^{th} amino acid leucine (L) is substituted with alanine (A) (L368A), and the 63^{rd} amino acid tyrosine (Y) is substituted with valine (V) (Y407V) (Hole). Conversely, the first fusion protein may be a first modified Fc region in which the 22^{nd} amino acid threonine (T) in the CH3 domain is substituted with serine (S) (T366S), the 24^{th} amino acid leucine (L) is substituted with alanine (A) (L368A), and the 63^{rd} amino acid tyrosine (Y) is substituted with valine (V) (Y407V) (Hole), and the second fusion protein may be a second modified Fc region in which the 22^{nd} amino acid threonine (T) in the CH3 domain is substituted with tryptophan (W) (T366W) (Knob).

The positions of the amino acid mutations are based on residues 224-330 of the CH3 region of human IgG1 amino acid sequence (UniProt No. P01857, SEQ ID NO: 61), and the numbers in parentheses referring to specific amino acid substitutions are according to the EU numbering scheme for antibody amino acid sequences. Even if additional mutations such as insertion, deletion, or substitution of amino acids occur in positions of the CH3 domain unrelated to the Knobs-into-Holes structure, variants corresponding to those positions based on the reference sequence could be used. Optionally, the Knobs-into-Holes structure may be introduced through other amino acid substitutions well known in the art. Such mutations are described in the prior art (Wei et al., Oncotarget 8(31): 51037-51049, 2017; Ridgway et al., Protein Eng. 9(7): 617-621, 1996; Carter, P., J. Immunol. Methods 48(1-2): 7-15, 2001). For example, a bispecific dimeric fusion protein may be generated by combining a Knob structure in which the 22^{nd} amino acid threonine (T) in the CH3 domain of the first fusion protein is substituted with tyrosine (Y) (T366Y), and a Hole structure in which the 63^{rd} amino acid tyrosine (Y) in the CH3 domain of the second fusion protein is substituted with threonine (T) (Y403T). The Knobs-into-Holes structure may also be formed in reverse, wherein the ole structure is introduced into the first fusion protein and the Knob structure into the second fusion protein.

As used herein, the term "KiHₛ₋ₛ" refers to a structure in which cysteine is introduced into the CH3 domain so that an intermolecular disulfide bond can be formed in the KiH structure, wherein the 10^{th} amino acid serine (S) of CH3 of the Knob Fc is additionally substituted with cysteine (C) (S354C), and the 5^{th} amino acid tyrosine (Y) of CH3 of the Hole Fc is additionally substituted with cysteine (C) (Y349C) (Merchant et al., Nat. Biotechnol. 16(7): 677-681, 1998).

As used herein, the term "HA-TF" refers to a technique of inducing heterodimerization by using hydrophobic/steric complementarity, in which the 20^{th} amino acid serine (S) of CH3 of the first modified Fc region is substituted with histidine (H) (S364H), the 61^{st} amino acid phenylalanine (F) is substituted with alanine (A) (F405A), the 5^{th} amino acid tyrosine (Y) of CH3 of the second modified Fc region is substituted with threonine (T) (Y349T), and the 50^{th} amino acid threonine (T) is substituted with phenylalanine (F) (T394F) (Moore et al., MAbs 3(6): 546-557, 2011).

As used herein, the term "ZW1" refers to a technique of inducing heterodimerization by using hydrophobic/steric complementarity, in which the 6^{th} amino acid threonine (T) of CH3 of the first modified Fc region is substituted with valine (V) (T350V), the 7^{th} amino acid leucine (L) is substituted with tyrosine (Y) (L351Y), the 61^{st} amino acid phenylalanine (F) is substituted with alanine (A) (F405A), the 63^{rd} amino acid tyrosine (Y) is substituted with valine (V) (Y407V), the 6^{th} amino acid threonine (T) of CH3 of the second modified Fc region is substituted with valine (V) (T350V), the 22^{nd} amino acid threonine (T) is substituted with tryptophan (W) (T366W), the 48^{th} amino acid lysine (K) is substituted with leucine (L) (K392L), and the 50^{th} amino acid threonine (T) is substituted with tryptophan (W) (T394W) (von Kreudenstein et al., MAbs 5(5): 646-654, 2013).

As used herein, the term "7.8.60" refers to a technique of inducing heterodimerization by using hydrophobic/steric complementarity and electrostatic complementarity, in which the 16^{th} amino acid lysine (K) of CH3 of the first modified Fc region is substituted with aspartic acid (D) (K360D), the 55^{th} amino acid aspartic acid (D) is substituted with methionine (M) (D399M), the 63^{rd} amino acid tyrosine (Y) is substituted with valine (V) (Y407V), the 1^{st} amino acid glutamic acid (E) of CH3 of the second modified Fc region is substituted with arginine (R) (E345R), the 3^{rd} amino acid glutamine (Q) is substituted with arginine (R) (Q347R), the 22^{nd} amino acid threonine (T) is substituted with valine (V) (T366V), and the 63^{rd} amino acid lysine (K) is substituted with valine (V) (K409V) (Leaver-Fay et al., Structure 24(4): 641-651, 2016).

As used herein, the term "DD-KK" refers to a technique of inducing heterodimerization by using electrostatic complementarity, in which the 65^{th} amino acid lysine (K) of CH3 of the first modified Fc region is substituted with aspartic acid (D) (K409D), the 48^{th} amino acid lysine (K) is substituted with aspartic acid (D) (K392D), the 55^{th} amino acid aspartic acid (D) of CH3 of the second modified Fc region is substituted with lysine (K) (D399K), and the 13^{th} amino acid glutamic acid (E) is substituted with lysine (K) (E356K) (Gunasekaran et al., J. Biol. Chem. 285(25): 19637-19646, 2010).

As used herein, the term "EW-RVT" refers to a technique of inducing heterodimerization by using hydrophobic/steric complementarity and long-range electrostatic interaction, in which the 16^{th} amino acid lysine (K) of CH3 of the first modified Fc region is substituted with glutamic acid (E) (K360E), the 65^{th} amino acid lysine (K) is substituted with tryptophan (W) (K409W), the 3^{rd} amino acid glutamine (Q) of CH3 of the second modified Fc region is substituted with arginine (R) (Q347R), the 55^{th} amino acid aspartic acid (D) is substituted with valine (V) (D399V), and the 61^{st} amino acid phenylalanine (F) is substituted with threonine (T) (F405T) (Choi et al., Mol. Cancer Ther. 12(12): 2748-2759, 2013).

As used herein, the term "EW-RVTs-s" refers to a structure in which cysteine is introduced into the CH3 domain so that an intermolecular disulfide bond can be formed in the EW-RVT structure, wherein the 5^{th} amino acid tyrosine (Y) of CH3 of the first modified Fc region of the EW-RVT is additionally substituted with cysteine (C) (Y349C), and the 10^{th} amino acid serine (S) of CH3 of the second modified Fc region is additionally substituted with cysteine (C) (S354C) (Choi et al., Mol. Immunol. 65(2): 377-383, 2015).

As used herein, the term "SEED" refers to a technique of inducing heterodimerization by utilizing hydrophobic/steric complementarity according to chain exchange between IgG and IgA, in which 45 residues derived from IgA are introduced into the CH3 of the first modified Fc region, and 57 residues derived from IgG1 are introduced into the CH3 of the second modified Fc region (Davis et al., Protein Eng, Des, Sel, 23(4): 195-202, 2010).

As used herein, the term "A107" refers to a technique of inducing heterodimerization by utilizing hydrophobic/steric complementarity and hydrogen bonding complementarity, in which the 26^{th} amino acid lysine (K) of CH3 of the first modified Fc region is substituted with glutamic acid (E) (K370E), the 69^{th} amino acid lysine (K) is substituted with tryptophan (W) (K409W), the 13^{th} amino acid glutamic acid (E) of CH3 of the second modified Fc region is substituted with asparagine (N) (E357N), the 55^{th} amino acid aspartic acid (D) is substituted with valine (V) (D399V), and the 61^{st} amino acid phenylalanine (F) is substituted with threonine (T) (F405T) (Choi et al., PLoS One 10(12): e0145349, 2015).

The hybrid antibody Fc region may be composed of the amino acid sequence selected from the group consisting of SEQ ID NOs: 12 to 16. In addition, the hybrid antibody Fc region may be further mutated so as not to cause undesirable side effects such as ADCC (antibody-dependent cell cytotoxicity) or CDC (complement-dependent cytotoxicity) upon *in vivo* administration. Such hybrid antibody Fc region may be those described in Korean Patent No. 897938. It may be a hybrid Fc region mutant (NTIG Knob) (SEQ NO ID: 15), wherein the 18^{th} amino acid, threonine (T), is substituted with glutamine (Q) and the 196^{th} amino acid methionine (M) is substituted with leucine (L) in the hybrid Fc region (Knob structure) presented by SEQ NO ID: 14. Or, it may be a hybrid Fc region mutant (NTIG Hole) (SEQ NO ID: 16), wherein the 18^{th} amino acid threonine (T) is substituted with glutamine (Q) and the 196^{th} amino acid methionine (M) is substituted with leucine (L) in the hybrid Fc region mutant (NTIG Hole) represented by SEQ ID NO: 13.

In the bispecific fusion protein, the GLP-2 analogue may be GLP-2, a GLP-2^{A2G} mutant, a GLP-2^{N16G_L17Q} mutant, a GLP-2^{A2G_N16G_L17Q} mutant, Glepaglutide, or GLP-2 analogue 10, and may be introduced with mutations such as amino acid substitutions, additions, or deletions for the purpose of reducing binding affinity to the GLP-2 receptor in order to achieve lower activity compared to wild-type GLP-2 activity. Alternatively, when using wild-type GLP-2, an approach to reduce binding affinity to the GLP-2 receptor may be employed by shortening the length relative to the GLP-1 analogue (for example, by inserting a longer linker peptide between the GLP-1 analogue and the Fc region). In this case, the length of the linker of the first fusion protein may be about 5 to 40 a.a. longer than that of the second fusion protein, and more specifically may be about 5 a.a., 6 a.a., 7 a.a., 8 a.a., 9 a.a., 10 a.a., 11 a.a., 12 a.a., 13 a.a., 14 a.a., 15 a.a., 16 a.a., 17 a.a., 18 a.a., 19 a.a., 20 a.a., 21 a.a., 22 a.a., 23 a.a., 24 a.a., 25 a.a., 26 a.a., 27 a.a., 28 a.a., 29 a.a., 30 a.a., 31 a.a., 32 a.a., 33 a.a., 34 a.a., 35 a.a., 36 a.a., 37 a.a., 38 a.a., 39 a.a., or 40 a.a., and may have a defined range by selecting any of the amino acid lengths as a lower limit and a greater value as an upper limit. In fact, through Examples 2 to 4 of the present invention, the present inventors confirmed that when the length of the GLP-1 analogue is designed to be longer than that of the GLP-2 analogue (by using a longer glycan linker peptide or by forming GLP-1 tandem repeats), the relative activity of GLP-2 is significantly decreased regardless of whether the GLP-2 analogue is mutated or not. Conversely, when a glycan linker is introduced into the GLP-2 analogue such that the fusion protein including the GLP-2 analogue becomes longer than the fusion protein including the GLP-1 analogue, it was confirmed that the relative activity of the GLP-2 analogue is greatly increased.

In the bispecific fusion protein, the GLP-2 analogue may comprise the amino acid sequence of any one of SEQ ID NOs: 17 to 22. The peptide composed of the amino acid sequence of SEQ ID NO: 18 is a human wild-type GLP-2 peptide, and the human GLP-2 mutant composed of the amino acid sequence of SEQ ID NO: 17 is one in which the second amino acid alanine is substituted with glycine (A2G mutant or GLP-2^{A2G}, hereinafter referred to as "GLP-2-2G" for convenience), also called Teduglutide. Meanwhile, the GLP-2 mutant composed of the amino acid sequence of SEQ ID NO: 19 is one in which, in addition to substitution of the second amino acid alanine (A) with glycine (G), the 16th amino acid asparagine (N) is substituted with glycine (G) and the 17th amino acid leucine (L) is substituted with glutamine (Q) (GLP-2^{A2G_N16G_L17Q}), which is known to inhibit dimerization or aggregate formation of GLP-2 during recombinant production while maintaining GLP-2 function (Baker et al., J. Mol. Recognit. 25: 155-164, 2012). Alternatively, in the wild-type GLP-2 peptide, substitution of the second amino acid alanine with glycine and the 17^{th} amino acid leucine with glutamine (A2G, L17Q, SEQ ID NO: 20) can exert the same function as the GLP-2 analogue composed of the amino acid sequence of SEQ ID NO: 19, and thus can be used as a GLP-2 analogue in the present invention.

In the bispecific fusion protein, the Glepaglutide may comprise the amino acid sequence set forth in SEQ ID NO: 21.

In the bispecific fusion protein, the GLP-2 analogue 10 may comprise the amino acid sequence set forth in SEQ ID NO: 22.

In the bispecific fusion protein, the first fusion protein may comprise the amino acid sequence selected from the group consisting of SEQ ID NOs: 23 to 25.

In the bispecific fusion protein, the second fusion protein may comprise the amino acid sequence selected from the group consisting of SEQ ID NOs: 26 to 28.

In the bispecific fusion protein, the first fusion protein may be of a Knob structure in which the 10^{th} amino acid of the CH3 domain of the hybrid Fc region, serine (S), is substituted with cysteine (C), and the 22^{nd} amino acid threonine (T) is substituted with tryptophan (W). The second fusion protein may be of a Hole structure in which the 5^{th} amino acid of the CH3 domain of the hybrid Fc region, tyrosine (Y), is substituted with cysteine (C), the 22^{nd} amino acid threonine (T) is substituted with serine (S), the 24^{th} amino acid leucine (L) is substituted with alanine (A), and the 63^{rd} amino acid tyrosine (Y) is substituted with valine (V). Conversely, the first fusion protein may be of a Hole structure with the above substitutions, and the second fusion protein may be of a Knob structure in which the 10^{th} amino acid serine (S) is substituted with cysteine (C), and the 22^{nd} amino acid threonine (T) is substituted with tryptophan (W).

Optionally, the first fusion protein may be one in which threonine (T) at position 22 of the CH3 domain in the hybrid Fc region is substituted with tyrosine (Y), and the second fusion protein may be one in which tyrosine (Y) at position 63 of the CH3 domain in the hybrid Fc region is substituted with threonine (T). Alternatively, the second fusion protein may be one in which tyrosine (Y) at position 63 of the CH3 domain in the hybrid Fc region is substituted with threonine (T), and the first fusion protein may be one in which tyrosine (Y) at position 63 of the CH3 domain in the hybrid Fc region is substituted with threonine (T). However, the substitution at position 63 is not based on the amino acid sequence of the CH3 domain of human IgG1 as set forth in SEQ ID No.: 61, but according to the numbering rule of IMGT (international ImMunoGeneTics information system) (Lefranc et al., Dev. Comp. Immunol., 27: 55-77, 2003), it may be designated as Y86T. In addition to the KiH structure, various other heterodimer-forming moieties as described above that can be introduced into the antibody Fc region to enable heterodimer formation may also be used.

In the bispecific fusion protein, one or more linker peptides may be inserted between the fusion partners of the fusion protein, i.e., between peptides or domains. Specifically, in the bispecific fusion protein comprising a GLP-1 analogue and a GLP-2 analogue, a linker peptide may be inserted between the GLP-1 analogue and the GLP-2 analogue. In the bispecific fusion protein generated by dimerization of the first and second fusion proteins, a linker peptide may be inserted between the GLP-1 analogue and the antibody Fc region within the first fusion protein, and similarly, a linker peptide may be inserted between the GLP-2 analogue and the antibody Fc region within the second fusion protein. In this case, the linker peptide may or may not contain an N-glycan attachment site. The purpose of introducing the N-glycan attachment site is to allow easier confirmation of heterodimer formation by creating a size difference between the first and second fusion proteins, and it has been confirmed that this does not significantly affect the binding affinity to GLP-1R or GLP-2R.

The linker peptide may be EPKSSDKTHTCPPCP (SEQ ID NO: 29), EPKSCDKTHTCPPCP (SEQ ID NO: 30), GGGGSGGGGSGGGGSEPKSSDKTHTCPPCP (SEQ ID NO: 31), GGGGSGGGGSGGGGSEPKSCDKTHTCPPCP (SEQ ID NO: 32), AKATTAPATTRNTGRGGEEKKKEKEKEEQEERETKTPECP (SEQ ID NO: 33), GGGGSGGGGSGGGGSEKEKEEQEERTHTCPPCP (SEQ ID NO: 34), GGGGSGGGGSGGGGSAKNTTAPATTRNTTRGGEEKKKEKEKEEQEERTHTCPPCP (SEQ ID NO: 35), AAGSGGGGGSGGGGSGGGGS (SEQ ID NO: 36), GGGGSGGGGSGGGGS (SEQ ID NO: 37), GGSGG (SEQ ID NO: 38), GGSGGSGGS (SEQ ID NO: 39), GGGSGG (SEQ ID NO: 40), (G₄S)ₙ (monomer: SEQ ID NO: 41, n is an integer from 1 to 10), (GGS)ₙ (n is an integer from 1 to 10), (GS)ₙ (n is an integer from 1 to 10), (GSSGGS)ₙ (monomer: SEQ ID NO: 42, n is an integer from 1 to 10), KESGSVSSEQLAQFRSLD (SEQ ID NO: 43), EGKSSGSGSESKST (SEQ ID NO: 44), GSAGSAAGSGEF (SEQ ID NO: 45), (EAAAK)ₙ (monomer: SEQ ID NO: 46, n is an integer from 1 to 10), CRRRRRREAEAC (SEQ ID NO: 47), A(EAAAK)₄ALEA(EAAAK)₄A (SEQ ID NO: 48), GGGGGGGG (SEQ ID NO: 49), GGGGGG (SEQ ID NO: 50), AEAAAKEAAAAKA (SEQ ID NO: 51), PAPAP (SEQ ID NO: 52), (Ala-Pro)n (n is an integer from 1 to 10), VSQTSKLTRAETVFPDV (SEQ ID NO: 53), PLGLWA (SEQ ID NO: 54), TRHRQPRGWE (SEQ ID NO: 55), AGNRVRRSVG (SEQ ID NO: 56), RRRRRRRR (SEQ ID NO: 57), GFLG (SEQ ID NO: 58), GSSGGSGSSGGSGGGDEADGSRGSQKAGVDE (SEQ ID NO: 59), or GSTSGSGKPGSGEGS (SEQ ID NO: 60).

According to one aspect of the present invention, there is provided a composition comprising the bispecific fusion protein.

According to another aspect of the present invention, there is provided a pharmaceutical composition for treating a metabolic disease, comprising one or more of the bispecific fusion proteins as an active ingredient.

In the pharmaceutical composition, the metabolic disease may be metabolic syndrome, obesity, diabetes, metabolic dysfunction-associated fatty liver disease, metabolic dysfunction-associated steatohepatitis, liver fibrosis, or cirrhosis. The diabetes may be type 1 diabetes or type 2 diabetes, and the liver fibrosis may be liver fibrosis caused by progression of chronic metabolic dysfunction-associated steatohepatitis.

According to another aspect of the present invention, one of the bispecific fusion proteins is provided for use in the treatment of a metabolic disease.

In the bispecific fusion protein, the metabolic disease may be metabolic syndrome, obesity, diabetes, metabolic dysfunction-associated fatty liver disease, metabolic dysfunction-associated steatohepatitis, liver fibrosis, or cirrhosis. The diabetes may be type 1 diabetes or type 2 diabetes, and the liver fibrosis may be liver fibrosis caused by progression of chronic metabolic dysfunction-associated steatohepatitis.

According to another aspect of the present invention, there is provided the use of one of the bispecific fusion proteins in the manufacture of a medicament for the treatment of a metabolic disease.

In the use, the metabolic disease may be metabolic syndrome, obesity, diabetes, metabolic dysfunction-associated fatty liver disease, metabolic dysfunction-associated steatohepatitis, liver fibrosis, or cirrhosis. The diabetes may be type 1 diabetes or type 2 diabetes, and the liver fibrosis may be liver fibrosis caused by progression of chronic metabolic dysfunction-associated steatohepatitis.

According to another aspect of the present invention, a method for treating a metabolic disease in a subject is provided, the method comprising administering to the subject a therapeutically effective amount of the bispecific fusion protein.

In the treatment method, the metabolic disease may be metabolic syndrome, obesity, diabetes, metabolic dysfunction-associated fatty liver disease, metabolic dysfunction-associated steatohepatitis, liver fibrosis, or cirrhosis. The diabetes may be type 1 diabetes or type 2 diabetes, and the liver fibrosis may be liver fibrosis caused by progression of chronic metabolic dysfunction-associated steatohepatitis.

Exendin-4, one of the GLP-1 receptor agonists, has already been proven in clinical trials to be effective in controlling blood glucose levels and body weight in patients with type 2 diabetes (DeFronzo et al., Diabetes Care 28: 1092-1100, 2005). Accordingly, the bispecific fusion protein according to one embodiment of the present invention can be used in the treatment of metabolic diseases such as obesity, diabetes, and metabolic syndrome.

The composition may further comprise a pharmaceutically acceptable carrier, and in addition to the carrier, may further comprise a pharmaceutically acceptable adjuvant, excipient, or diluent.

As used herein, the term "pharmaceutically acceptable" refers to a composition that is physiologically acceptable and does not cause, when administered to a human, an allergic reaction such as gastrointestinal disturbance or dizziness, or a similar reaction. Examples of the carrier, excipient, and diluent include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. In addition, fillers, anti-caking agents, lubricants, wetting agents, flavoring agents, emulsifiers, and preservatives may also be included.

In one embodiment of the present invention, the pharmaceutical composition may be formulated using methods known in the art so that, upon administration to a mammal, the active ingredient can be rapidly released, or released in a sustained or delayed manner. The formulation may include powders, granules, tablets, emulsions, syrups, aerosols, soft or hard gelatin capsules, sterile injectable solutions, or sterile powders.

The composition according to one embodiment of the present invention may be administered through various routes, for example, orally, non-orally such as by suppository, transdermally, intravenously, intraperitoneally, intramuscularly, intralesionally, intranasally, or intrathecally, and may also be administered using an implant device for sustained, continuous, or repeated release. The number of administrations may be once per day or divided into multiple doses within a day within a desired range, and the duration of administration is not particularly limited.

The composition according to one embodiment of the present invention may be formulated in an appropriate form together with pharmaceutically acceptable carriers commonly used. Examples of pharmaceutically acceptable carriers include carriers for non-oral administration such as water, suitable oils, saline, aqueous glucose, and glycols, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. Further, depending on the method of administration or formulation, the composition according to the present invention may, if necessary, suitably include suspending agents, solubilizers, stabilizers, isotonic agents, preservatives, anti-adsorption agents, surfactants, diluents, excipients, pH regulators, analgesics, buffers, or antioxidants. Pharmaceutically acceptable carriers and preparations suitable for the present invention, including those exemplified above, are described in detail in the literature [Remington's Pharmaceutical Sciences, latest edition].

The dosage of the composition for a patient varies depending on many factors, including the patient's renal function, body surface area, age, the specific compound administered, sex, timing and route of administration, general health, and other drugs administered simultaneously. The pharmaceutically active protein may be administered in an amount of 100 ng/kg body weight to 10 mg/kg body weight, preferably 1 to 500 µg/kg body weight, and most preferably 5 to 50 µg/kg body weight, with the dosage being adjustable in consideration of the above factors.

As used herein, the term "therapeutically effective amount" means an amount sufficient to treat a disease with a reasonable benefit/risk ratio applicable to medical treatment, and the effective dose level may be determined depending on factors such as the type and severity of the disorder, age, sex, activity of the drug, sensitivity to the drug, timing of administration, route of administration and rate of excretion, duration of treatment, concomitant drugs, and other factors well known in the medical field. The therapeutically effective amount of the composition of the present invention may be 0.1 mg/kg to 1 g/kg, more preferably 1 mg/kg to 500 mg/kg, but the effective dosage may be suitably adjusted depending on the patient's age, sex, and condition.

Between two or more proteins or domains, a linker peptide generally having a flexible structure may be inserted. The linker peptide may include EPKSSDKTHTCPPCP (SEQ ID NO: 29), EPKSCDKTHTCPPCP (SEQ ID NO: 30), GGGGSGGGGSGGGGSEPKSSDKTHTCPPCP (SEQ ID NO: 31), GGGGSGGGGSGGGGSEPKSCDKTHTCPPCP (SEQ ID NO: 32), AKATTAPATTRNTGRGGEEKKKEKEKEEQEERETKTPECP (SEQ ID NO: 33), GGGGSGGGGSGGGGSEKEKEEQEERTHTCPPCP (SEQ ID NO: 34), GGGGSGGGGSGGGGSAKNTTAPATTRNTTRGGEEKKKEKEKEEQEERTHTCPPCP (SEQ ID NO: 35), AAGSGGGGGSGGGGSGGGGS (SEQ ID NO: 36), GGGGSGGGGSGGGGS (SEQ ID NO: 37), GGSGG (SEQ ID NO: 38), GGSGGSGGS (SEQ ID NO: 39), GGGSGG (SEQ ID NO: 40), (G₄S)ₙ (monomer: SEQ ID NO: 41, n is an integer from 1 to 10), (GGS)n (n is an integer from 1 to 10), (GS)ₙ (n is an integer from 1 to 10), (GSSGGS)ₙ (monomer: SEQ ID NO: 42, n is an integer from 1 to 10), KESGSVSSEQLAQFRSLD (SEQ ID NO: 43), EGKSSGSGSESKST (SEQ ID NO: 44), GSAGSAAGSGEF (SEQ ID NO: 45), (EAAAK)ₙ (monomer: SEQ ID NO: 46, n is an integer from 1 to 10), CRRRRRREAEAC (SEQ ID NO: 47), A(EAAAK)₄ALEA(EAAAK)₄A (SEQ ID NO: 48), GGGGGGGG (SEQ ID NO: 49), GGGGGG (SEQ ID NO: 50), AEAAAKEAAAAKA (SEQ ID NO: 51), PAPAP (SEQ ID NO: 52), (Ala-Pro)ₙ (n is an integer from 1 to 10), VSQTSKLTRAETVFPDV (SEQ ID NO: 53), PLGLWA (SEQ ID NO: 54), TRHRQPRGWE (SEQ ID NO: 55), AGNRVRRSVG (SEQ ID NO: 56), RRRRRRRR (SEQ ID NO: 57), GFLG (SEQ ID NO: 58), GSSGGSGSSGGSGGGDEADGSRGSQKAGVDE (SEQ ID NO: 59), or GSTSGSGKPGSGEGS (SEQ ID NO: 60).

According to another aspect of the present invention, the bispecific fusion protein can be produced by recombinantly expressing a recombinant expression vector introduced into a host cell, wherein the recombinant expression vector comprises the first gene construct including a polynucleotide encoding the first fusion protein and the second gene construct including a polynucleotide encoding the second fusion protein.

In this case, the first gene construct and the second gene construct may be inserted and expressed in a single expression vector, or each may be inserted and expressed in two separate expression vectors. In the former case, the vector may be designed such that the two gene constructs are operably linked to two separate regulatory sequences respectively, or, both gene constructs are operably linked to a single regulatory sequence and linked to each other by an internal ribosome entry site (IRES).

As used herein, the term "operably linked to" means that the target nucleic acid sequence (for example, in an *in vitro* transcription/translation system or in a host cell) is linked to the regulatory sequence in such a way that its expression can occur.

The term "regulatory sequence" refers to promoters, enhancers, and other regulatory elements (e.g., polyadenylation signals). The regulatory sequence includes those that direct constitutive expression of the target nucleic acid in many host cells, those that direct expression of the target nucleic acid only in particular tissue cells (e.g., tissue-specific regulatory sequences), and those that direct inducible expression in response to particular signals (e.g., inducible regulatory sequences). It will be understood by those skilled in the art that the design of the expression vector may vary depending on factors such as the choice of host cell to be transformed and the desired level of protein expression. The expression vector of the present invention may be introduced into a host cell to express the fusion protein. Regulatory sequences that allow expression in eukaryotic and prokaryotic cells are well known to those skilled in the art. As described above, these usually include regulatory sequences responsible for initiation of transcription and, optionally, poly-A signals responsible for termination and stabilization of the transcript. Additional regulatory sequences may include translational enhancers and/or natural-composite or heterologous promoter regions in addition to transcriptional regulators. For example, possible regulatory sequences that allow expression in mammalian host cells include CMV-HSV thymidine kinase promoter, SV40, RSV promoter (Rous sarcoma virus), human elongation factor 1α promoter, glucocorticoid-inducible MMTV promoter (Moloney murine tumor virus), metallothionein-inducible or tetracycline-inducible promoters, or enhancers such as CMV enhancer or SV40 enhancer. For expression in neuronal cells, it is considered that neurofilament promoter, PGDF promoter, NSE promoter, PrP promoter, or thy-1 promoter may be used. These promoters are known in the art and are described in the literature (Charron, J. Biol. Chem. 270: 25739-25745, 1995). For expression in prokaryotic cells, numerous promoters including lac promoter, tac promoter, or trp promoter have been disclosed. In addition to factors that can initiate transcription, the regulatory sequence may further include transcription termination signals such as SV40 poly-A site or TK poly-A site downstream of the polynucleotide according to one embodiment of the present invention. In this document, suitable expression vectors are known in the art, examples of which include the Okayama-Berg cDNA expression vector pcDV1 (Pharmacia), pRc/CMV, pcDNA1, pcDNA3 (Invitrogen), pSPORT1 (GIBCO BRL), pGX-27 (Patent No. 1442254), pX (Pagano et al., Science 255: 1144-1147, 1992), yeast two-hybrid vectors such as pEG202 and dpJG4-5 (Gyuris et al., Cell 75: 791-803, 1995), or prokaryotic expression vectors such as lambda gt11 or pGEX (Amersham Pharmacia). In addition to the nucleic acid molecules of the present invention, the vector may further include a polynucleotide encoding a secretion signal. Such secretion signals are well known to those skilled in the art. Further, depending on the expression system used, a leader sequence that can direct the fusion protein to a cellular compartment may be combined with the coding sequence of the polynucleotide according to one embodiment of the present invention, preferably a leader sequence that can directly secrete the translated protein into the pericellular region or extracellular medium.

Also, the vector of the present invention may be produced, for example, by standard recombinant DNA techniques. The recombinant DNA techniques include, for instance, blunt-end and cohesive-end ligation, restriction enzyme treatment to provide suitable ends, removal of phosphate groups by alkaline phosphatase treatment to prevent undesirable ligation, and enzymatic joining by T4 DNA ligase. The vector of the present invention can be produced by recombining a DNA that encodes a signal peptide obtained by chemical synthesis or genetic recombination technology, and a DNA that encodes the bispecific fusion protein of the present invention into a vector containing an appropriate regulatory sequence. The vector containing such regulatory sequences can be purchased or manufactured commercially, and in one embodiment of the present invention, pBispecific backbone vector (Genexine, Inc., Republic of Korea) or pAD15 vector was used as the backbone vector.

The expression vector may further include a polynucleotide encoding a secretion signal sequence, which directs the secretion of the recombinant protein expressed in the cell to the extracellular region. The secretion signal sequence may be, for example, a tissue plasminogen activator (tPA) signal sequence, a herpes simplex virus glycoprotein Ds (HSV gDs) signal sequence, or a growth hormone signal sequence.

In one embodiment of the present invention, the expression vector may be an expression vector that enables the protein to be expressed in a host cell, and the expression vector may be in any form, including a plasmid vector, a viral vector, a cosmid vector, a phagemid vector, or an artificial human chromosome.

### MODE FOR CARRYING OUT THE INVENTION

Hereinafter, the present invention will be described in greater detail through Examples and Experimental Examples. However, the present invention is not limited to the following Examples and Experimental Examples, but may be embodied in various different forms. The following Examples and Experimental Examples are provided to ensure a complete disclosure of the present invention and to fully inform those skilled in the art of the scope of the invention.

### Example: Design of Bispecific Fusion Proteins

The present inventors designed various bispecific fusion proteins comprising both a GLP-1 analogue and a GLP-2 analogue, as illustrated in FIG. 1 and Table 1.

In designing the bispecific fusion proteins comprising a GLP-1 analogue and a GLP-2 analogue, GLP-1/Exendin 4 hybrid (hereinafter, for convenience, abbreviated as "GLP-1/Ex4 Hyb") and their tandem repeats, and GLP-2 analogues (GLP-2-2G and mutants having substitutions at positions 16 and 17 (GLP-2-2G mutant)) were used to regulate the activities of the GLP-1 analogue and the GLP-2 analogue. For ease of purification, a glycan linker was employed in either one of the first fusion protein comprising a GLP-1 analogue and the second fusion protein comprising a GLP-2 analogues, or no glycan linker was employed in either of them.

**[Table 1]**

| Example | Name | GLP-1/2 Analog (SEQ ID NO) | Linker (SEQ ID NO) | Fc Region | Overall Composition (SEQ ID NO) |
|---|---|---|---|---|---|
| Comparative Example | MG12-5 | GLP-1/Ex4 Hyb (5) | None (34) | knob | First fusion protein (23) |
| | | 2. GLP-2-2G (17) | None (34) | hole | Second fusion protein (26) |
| 1 | MG12-6 | GLP-2-2G (17) | Glycan (35) | hole | Second fusion protein (27) |
| | | 2. GLP-1/Ex4 Hyb (5) | None (34) | knob | First fusion protein (23) |
| 2 | MG12-7 | GLP-1/Ex4 Hyb (5) | Glycan (35) | knob | First fusion protein (24) |
| | | 2. GLP-2-2G mutant (19) | None (31) | hole | Second fusion protein (28) |
| 3 | MG12-8 | GLP-1/Ex4 Hyb (5) | None (31) | knob | First fusion protein (25) |
| | | 2. GLP-2-2G mutant (19) | None (31) | hole | Second fusion protein (28) |
| 4 | MG12-9 | GLP-1/Ex4 Hyb (5) | None (31) | knob | First fusion protein (25) |
| | | 2. GLP-2-2G (17) | None (34) | hole | Second fusion protein (26) |

In addition, in order to preferentially generate heterodimers in the case of the above-described bispecific fusion proteins, the Knobs-into-holes (KiH) technology was applied. The first fusion protein may be one in which the serine (S) at the 10^{th} amino acid of the CH3 domain of the hybrid Fc region is substituted with cysteine (C), and the threonine (T) at the 22^{nd} amino acid is substituted with tryptophan (W) (Knob). The second fusion protein may be one in which the tyrosine (Y) at the 5^{th} position of the CH3 domain of the Fc region is substituted with cysteine (C), the threonine (T) at the 22^{nd} position is substituted with serine (S), the leucine (L) at the 24^{th} position is substituted with alanine (A), and the tyrosine (Y) at the 63^{rd} position is substituted with valine (V) (Hole). In this case, the positions of the amino acids where the mutations occur are based on the reference sequence (the amino acid sequence of the IgG1 CH3 domain of SEQ ID NO: 61). Even if additional mutations, such as addition, deletion, or substitution of amino acids, occur in regions of the CH3 domain unrelated to the Knobs-into-Holes structure, the mutated amino acid corresponding to the position based on the reference sequence may be used. Optionally, the Knobs-into-holes structure may be introduced through other amino acid substitutions well known in the art. Such variations are described in prior literature (Wei et al., Oncotarget 2017, 8(31): 51037-51049; Ridgway et al., Protein Eng. 1996, 9(7): 617-621; Carter, P., J. Immunol. Methods 2001, 48(1-2): 7-15; Merchant et al., Nat. Biotechnol. 1998, 16(7): 677-681). Such optional variants may include a bispecific dimeric fusion protein formed through the combination of a Knob structure, in which threonine at the 22^{nd} position of the CH3 domain of the first fusion protein is substituted with tyrosine, and a Hole structure, in which tyrosine at the 63^{rd} position of the CH3 domain of the second fusion protein is substituted with threonine. Conversely, the Knobs-into-holes structure may be formed by introducing the Hole structure into the first fusion protein and the Knob structure into the second fusion protein. After synthesizing gene constructs encoding the first and second fusion proteins of the bispecific fusion protein of the comparative example and the bispecific fusion proteins of Examples 1 to 4, the plasmid DNA was transfected into HEK293F cells using the N293F vector system (Y-Biologics) for transient expression in mammalian cells. For nucleic acid preparation, 25 µg of plasmid DNA was mixed with 3 ml of medium, followed by the addition and mixing of 25 µg of 2 mg/ml PEI (polyethylenimine, PolyPlus, USA). The reaction mixture was allowed to stand at room temperature for 15 minutes and then added to 80-1,000 ml of culture medium containing cells cultured at 1×10⁶ cells/ml. The culture was incubated at 120 rpm, 37°C, and 8% CO₂ for 24 hours. Twenty-four hours after DNA transfection, a nutrient supplement (Soytone, BD, USA) was added to a final concentration of 10 g/L. One day after transfection, the temperature was lowered to 32°C, and incubation was continued until day 7.

The supernatant obtained from the culture was purified through a Protein A column and a secondary column to yield the bispecific fusion protein of the comparative example (designated "MG12-5") and the bispecific fusion proteins of Examples 1 to 4 (designated "MG12-6," "MG12-7," "MG12-8," and "MG12-9," respectively). These were diluted with 4× LDS sample buffer and sterile water for injection to prepare final concentrations of 3-10 µg/20 µL. For reduced samples, each substance to be analyzed was diluted with 4× LDS sample buffer, 10× reducing agent, and sterile water for injection to a final concentration of 3-10 µg/20 µL, followed by heating at 95°C for 10 minutes. The prepared samples were loaded, 20 µL per well, into gels mounted on a pre-installed electrophoresis apparatus. For the size marker, 3-5 µL per well was loaded. Electrophoresis was carried out at 120 V for 90 minutes. After electrophoresis, the gel was separated, stained using staining and destaining solutions, and the results were analyzed.

As shown in FIGs. 2a to 2d, all bispecific fusion proteins were observed between 50-75 kDa under non-reducing (NR) conditions, and at approximately 37 kDa under reducing (R) conditions.

In conclusion, SDS-PAGE confirmed that all bispecific fusion proteins according to the Examples of the present invention could be properly produced and purified. In particular, as demonstrated by the comparison of MG12-5 with the GLP-2-Fc homodimer, the application of the KiH structure to bispecific fusion proteins significantly reduced the formation of monomeric impurities compared with cases without such application.

### Experimental Example 1: In vitro Activity Assay of GLP-1 and GLP-2

The present inventors investigated the *in vitro* activity of GLP-1 and GLP-2 of the bispecific fusion proteins prepared in the above Examples. Specifically, to evaluate the activity of the GLP-1 receptor, CHO-K1 cells (DiscoverX) stably expressing the GLP-1 receptor were used. The GLP-1R-expressing CHO-K1 cells were subcultured, dispensed into 96-well white plates at a density of 5×10⁵ cells/mL in 100 µL per well, and incubated at 37°C in a 5% CO₂ incubator for 20-24 hours. After confirming that the cells were stably attached, the test drugs were prepared at three times the final concentration. The culture medium in the plate was removed, and 30 µL of assay reagent and 15 µL of GLP-1-related drugs were added. Following DIRANF treatment, the solution was incubated at 37°C for 30 minutes, then 75 µL of cell lysis solution and assay reagent were added. It was reacted for 1 hour at room temperature, followed by induction of fluorescence for 4-6 hours. By this process, agonists of the GLP-1 receptor bound to and stimulated the CHO-K1 cells, resulting in an increase in cAMP. The luciferase activity corresponding to this increase was measured as bioluminescence using the Spectramax M5 instrument, thereby evaluating the activity of the drugs.

For evaluation of GLP-2 receptor activity, CHO-K1 cells (DiscoverX) stably expressing the GLP-2 receptor were used. Except for the use of GLP-2R-expressing CHO-K1 cells, the method was the same as described above for evaluating GLP-2 activity. The activity of the comparative example MG12-5 was referenced from the values disclosed in Korean Patent No. 10-2349718.

As shown in Tables 2 and 3, with respect to GLP-1 activity, the comparative example MG12-5 exhibited 35% activity relative to wild-type GLP-1, whereas the bispecific fusion proteins of Examples 1 to 3 of the present invention exhibited at least 40% or more of the activity of wild-type GLP-1 peptide, confirming that they showed activity comparable to or higher than wild-type GLP-1. In particular, Example 2 exhibited high activity of more than 150% relative to wild-type GLP-1. In addition, with respect to GLP-2 activity, unlike the comparative example MG12-5 which showed 59% activity of wild-type GLP-2, all of the bispecific fusion proteins of Examples 1 to 3 exhibited less than 50% activity, and in particular, the bispecific fusion proteins of Examples 2 and 3 showed very low activities of about 2% and 7% relative to wild-type GLP-2, respectively.

**[Table 2]**

| ***In vitro* GLP-1 activity analysis of bispecific fusion proteins according to the Examples of the present invention** | | | | | |
|---|---|---|---|---|---|
| | | hGLP-1 | Example 1 | Example 2 | Example 3 |
| GLP-1 activity (Set1) | EC₅₀ (nM) | 0.69 | 1.57 | 0.35 | 0.81 |
| | Relative activity compared with hGLP-1 peptide (%) | 100 | 44 | 194 | 85 |
| GLP-1 activity (Set2) | EC₅₀ (nM) | 0.89 | 1.12 | 0.57 | 0.71 |
| | Relative activity compared with hGLP-1 peptide (%) | 100 | 79 | 156 | 125 |

**[Table 3]**

| ***In vitro* GLP-2 activity analysis of bispecific fusion proteins according to the Examples of the present invention** | | | | | |
|---|---|---|---|---|---|
| | | hGLP-2 | Example 1 | Example 2 | Example 3 |
| GLP-2 activity | EC₅₀ (nM) | 1.18 | 2.42 | 50.8 | 19 |
| | Relative activity compared with hGLP-2 peptide (%) | 100 | 49 | 2 | 6 |

### Experimental Example 2: Analysis of Weight Reduction Effect

### 2-1: Analysis of effect upon single administration

The present inventors evaluated the weight reduction effect of a single administration of MG12-5, used as a comparative group, and MG12-8, according to one embodiment of the present invention. Specifically, normal 7-week-old male C57BL/6N mice were obtained and acclimated for one week with free access to food and water. After measuring the body weight of the experimental animals, the mice were randomly assigned into groups so that the average body weight was similar across groups. And then, MG12-5 (100 nmol/5 mL/kg), MG12-8 (100 nmol/5 mL/kg), each prepared in MG12 formulation buffer, and vehicle (without drug) as a negative control were subcutaneously injected into the flank of the animals, respectively. Three days after drug administration, the body weight of the mice was measured to evaluate the weight reduction effect induced by the drug (FIG. 3a).

As a result, as shown in FIG. 3b, no body weight change or even an increase was observed in the vehicle-administered group following injection. In contrast, in the control group administered with MG12-5, the body weight was maintained at a level very similar to that before drug administration, whereas in the group administered with MG12-8 according to one embodiment of the present invention, the body weight was reduced by approximately 5%. From this, it was confirmed that for the bispecific fusion protein comprising both GLP-1 analogue and GLP-2 analogue, the GLP-1 activity should be at least 40% relative activity compared to wild-type GLP-1 peptide, or the GLP-2 activity should be 50% or less relative activity compared to wild-type GLP-2 peptide.

### 2-2: Analysis of effect upon repeated administration

Subsequently, the present inventors evaluated the weight reduction efficacy of repeated administration of MG12-8 according to one embodiment of the present invention, compared with the comparative example MG12-5. Specifically, normal seven-week-old male C57BL/6J mice were obtained and acclimated for one week under free access to food and water. Thereafter, the body weights of the mice were measured, and the mice were randomly assigned to groups such that the average body weights were similar. MG12-5 (30 nmol/5 mL/kg) and MG12-8 (30 nmol/5 mL/kg) prepared from MG12 formulation buffer, and vehicle without any drug inside were administered subcutaneously into the flank of the mice once every two days for a total of seven times over two weeks. Thereafter, body weights were recorded every two days starting from the first day of drug administration, and the body weight change relative to the pre-administration baseline was expressed as a percentage (%).

As a result, as shown in FIG. 3d, body weight reduction by drug administration was observed in both the groups administrated with the comparative example and the bispecific fusion protein according to one embodiment of the present invention, unlike in the vehicle-administered group. However, in the case of repeated administration of MG12-5 (comparative example), it was confirmed that this weight reduction effect gradually returned to normal levels. In contrast, in the group administered with MG12-8 according to one embodiment of the present invention, an additional ~5% weight reduction effect was observed compared with MG12-5, and the weight reduction by repeated administration was maintained relative to MG12-5.

Meanwhile, it is known that GLP-2R agonists, which are mainly used for the treatment of short bowel syndrome, exhibit gallbladder enlargement as a side effect. Accordingly, in the case of bispecific fusion proteins comprising both a GLP-1R agonist and a GLP-2R agonist as in the present invention, if the GLP-2R agonist activity is too strong, such side effects may occur. Therefore, to confirm the gallbladder volume after the last administration, the mice were sacrificed the day after the final drug administration, and photographs were taken by placing a ruler next to the gallbladder to measure its volume.

As a result, as shown in FIG. 3e, observation of gallbladder enlargement upon repeated drug administration revealed that the gallbladder volume increased 2.3-fold in the group administered with the comparative example MG12-5 compared with the vehicle-administered group. In contrast, in the group administered with MG12-8 according to one embodiment of the present invention, there was no statistically significant difference from the vehicle-administered group. From this, it was confirmed that the GLP-2 activity of the bispecific fusion protein of the present invention must be less than 50% relative activity compared with wild-type GLP-2 peptide in order not to exhibit toxicity.

### Experimental Example 3: Analysis of therapeutic effect on metabolic dysfunction-associated steatohepatitis with intestinal leakage

Subsequently, the present inventors investigated the weight reduction and regulation of intestinal leakage in a metabolic dysfunction-associated steatohepatitis (MASH) animal model with intestinal leakage, using Liraglutide as a comparative control and MG12-8 according to one embodiment of the present invention. Specifically, twenty-week-old male C57BL/6J mice were obtained, and overweight was induced by feeding a choline-deficient high-fat diet (CD-HFD). In addition, intestinal leakage was induced by cyclically administering dextran sulfate sodium (DSS) at a low concentration of 1% for 7 days followed by 10 days of regular drinking water. After two weeks from the initiation of CD-HFD feeding, Liraglutide and MG12-8 were subcutaneously administered at their respective prescribed dosages and dosing intervals. Two weeks after the first administration, body weight was measured (FIG. 4a). To assess intestinal leakage, a fluorescein isothiocyanate (FITC)-Dextran (4 kDa) solution was administered orally 4 hours before sacrifice, after which the mice were fasted. The amount of FITC-Dextran translocated into the blood was then evaluated by measuring fluorescence intensity in serum.

As a result, as shown in FIG. 4b, the final body weight of the vehicle-administered group was approximately 37 g, which is more than 20% higher than the normal body weight range of 30 g. In contrast, both the Liraglutide-administered group and the group administered with MG12-8 according to one embodiment of the present invention exhibited reduced body weights compared with the vehicle-administered group, with MG12-8 showing the most effective weight-reducing efficacy.

Furthermore, analysis of the effect on intestinal leakage revealed, as shown in FIG. 4c, that MG12-8 according to one embodiment of the present invention exhibited superior intestinal leakage suppression activity compared with the control group. On the other hand, Liraglutide did not exhibit intestinal leakage suppression activity relative to the control. Moreover, considering that Liraglutide was administered at a relatively high dose of 55 nmol/kg twice daily, whereas MG12-8 according to one embodiment of the present invention was administered at a lower dose of 15 nmol/kg only three times per week (more than four times longer dosing interval than Liraglutide), it is evident that MG12-8 is a more advantageous substance in terms of patient convenience.

### Experimental Example 4: In vivo analysis of GLP-1 activity

The present inventors analyzed *in vivo* GLP-1 activity of MG12-8 according to one embodiment of the present invention. For this purpose, four MG12-8 samples exhibiting different *in vitro* GLP-1 activities were obtained through RP-HPLC analysis. The RP-HPLC results showed differences in the peak areas of Peak 1 and Peak 3. In Sample 1, the area of Peak 1 was small, and as the sample number increased, the area of Peak 1 increased, whereas the area of Peak 3 decreased (Table 4). These four samples, each having different ratios of Peak 1 to Peak 3 area, were subjected to *in vitro* GLP-1 activity analysis using HEK-GLP-1R cells (eEnzyme), which stably express GLP-1R. Specifically, the HEK-GLP-1R cells were subcultured and seeded into 96-well assay plates at a density of 3×10⁵ cells/mL, 100 µL per well, and incubated in a 37 °C, 5% CO₂ incubator for 20-24 hours. After confirming stable cell attachment, the drugs were prepared at five times the final concentration, the culture medium in the plates was removed, and appropriate amounts of test reagents and GLP-1-related drugs were added. After a 2-hour incubation with the drugs, the amount of increased cAMP was evaluated by measuring bioluminescence using the kit-provided solution and a SpectraMax M5 instrument, thereby assessing the drug activity.

**[Table 4]**

| **RP-HPLC Analysis results of different samples** | | |
|---|---|---|
| Sample | First peak (%) | Third peak (%) |
| #1 | 11.9 | 71.3 |
| #2 | 34.0 | 54.0 |
| #3 | 57.9 | 33.6 |
| #4 | 67.5 | 24.9 |

As shown in FIG. 5a, the relative GLP-1 activity was found to be 100% for Sample #1, approximately 200% for Sample #2, approximately 300% for Sample #3, and approximately 400% for Sample #4. Subsequently, the present inventors investigated the *in vivo* GLP-1 activity of the four samples exhibiting different *in vitro* GLP-1 activities as described above by conducting animal experiments. Specifically, the present inventors performed an intraperitoneal glucose tolerance test (IPGTT). Eighteen hours prior to the initiation of IPGTT, the respective drug was administered, and both feed and bedding were removed to initiate fasting. Eighteen hours after drug administration, a 20% glucose solution was intraperitoneally injected, and changes in blood glucose levels before and after injection were monitored over time (FIG. 5b).

As shown in FIG. 5c, samples exhibiting *in vitro* GLP-1 activity of 100-300% showed comparable *in vivo* blood glucose suppression efficacy. In contrast, the sample with *400% in vitro* GLP-1 activity demonstrated stronger blood glucose suppression efficacy compared to the 100% sample. From this, it was confirmed that the bispecific fusion protein of the present invention exhibiting 100-300% GLP-1 activity relative to wild-type GLP-1 peptide under *in vitro* conditions exhibited similar efficacy under *in vivo* conditions.

### Experimental Example 5: Analysis of Anti-obesity Effects in a High-Fat Diet Model

### 5-1: Comparative Efficacy Analysis with Conventional GLP-1R/GLP-2R Dual Agonist

The present inventors further sought to compare the anti-obesity efficacy of the bispecific fusion protein of the present invention with that of a conventional GLP-1R/GLP-2R dual agonist. Specifically, for efficacy comparison with a known GLP-1R/GLP-2R dual agonist peptide substance (Dapiglutide mimetic), body weight reduction and body composition changes were analyzed upon treatment with 30 nmol/kg of Dapiglutide mimetic and 15 or 30 nmol/kg of MG12-8 (hereinafter referred to as "MG12" for convenience). Body composition was analyzed using a Minispec LF50 (Bruker Biospin, BCA LF50), measuring total body fat mass and lean mass (including muscle and bone). To evaluate the changes induced by drug administration, the rate of change in body composition before and after two weeks of drug administration was determined. For this purpose, 5-week-old male C57BL/6N mice were obtained, acclimated for one week, and subsequently subjected to a high-fat diet (HFD, 60%) to induce diet-induced obesity (DIO). Sixteen weeks after initiation of the HFD, appropriately prepared Dapiglutide mimetic and the bispecific fusion protein of the present invention (MG12) were administered subcutaneously into the flank of mice once every two days.

As shown in the left graph of FIG. 6a, analysis of body weight and body composition changes before and after two weeks of drug administration revealed that treatment with 30 nmol/kg of Dapiglutide mimetic resulted in approximately 5% body weight reduction. In contrast, treatment with 15 nmol/kg of MG12 resulted in 13% weight reduction, while 30 nmol/kg of MG12 resulted in approximately 20% weight reduction.

Furthermore, measurement of the fat-to-lean tissue weight ratio after sacrifice at the end of the experiment showed, as illustrated in the right graph of FIG. 6a, that both the normal diet group and the high-fat diet group exhibited similar positive fat mass increase rates. However, in both the Dapiglutide mimetic-administered group and the group administered with the bispecific fusion protein according to one embodiment of the present invention, a reduction in fat mass within body composition was observed. Notably, while the Dapiglutide mimetic-administered group showed approximately 6% reduction, the group admnistered with 15 nmol/kg of MG12 showed an 18% reduction, and the group treated with 30 nmol/kg of MG12 showed a 35% reduction in fat ratio, confirming that the substance of the present invention exhibits superior anti-obesity efficacy compared to existing agents.

### 5-2: Analysis of Dose-Dependent Effects

The present inventors further investigated whether the *in vivo* effects of the bispecific fusion protein (MG12) according to one embodiment of the present invention are exhibited in a dose-dependent manner. To this end, various concentrations of the bispecific protein were used to analyze the weight loss effect in high-fat diet (HFD)-induced obese animal models. Specifically, the present inventors obtained 5-week-old normal C57BL/6 male mice, acclimated them for one week, and then induced diet-induced obesity (DIO) by administering a 60% HFD. After 16 weeks from the start of HFD, MG12 was formulated at 15, 30, and 60 nmol/5 mL/kg using MG12 formulation buffer, and administered subcutaneously into the abdominal region once every two days. The mice were randomly grouped so that the average body weight was similar among groups, and body weight changes after drug administration were expressed as percentages.

As shown in FIG. 6b, both the normal diet group and the HFD group showed a tendency to maintain or increase body weight over two weeks. However, in the groups administered with MG12, weight loss efficacy of approximately 13-25% was observed depending on the increasing concentration, confirming that the weight loss effect appeared in a dose-dependent manner.

Subsequently, the present inventors examined whether the effect of the bispecific fusion protein according to one embodiment of the present invention was similar to that of simple co-administration of GLP-1 and GLP-2 peptides. To this end, the present inventors separately produced GLP-1-Fc and GLP-2-Fc, which are fusion partners of the bispecific fusion protein according to one embodiment of the present invention, and compared the body weight changes between simple co-administration of the two (30 nmol/kg + 30 nmol/kg) and administration of the bispecific fusion protein (MG12) at the same molar concentration (30 nmol/kg).

As shown in FIG. 6c, compared to the simple combination of GLP-1-Fc and GLP-2-Fc, MG12 at the same dosage improved weight loss by about 5%.

### 5-3: Analysis of Fat-Specific Weight Reduction Effects

The present inventors further compared the fat-specific weight reduction effects of the bispecific fusion protein according to one embodiment of the present invention with Tirzepatide mimetic, a GLP-1R/GIPR peptide dual agonist currently used as an anti-obesity therapeutic. Specifically, the present inventors obtained 5-week-old normal C57BL/6 male mice, acclimated them for one week, and induced DIO by administering a 60% HFD. After 16 weeks from the start of HFD, Tirzepatide mimetic and MG12 were prepared using MG12 formulation buffer at 15 and 60 nmol/5 mL/kg, respectively, and administered subcutaneously. Body composition analysis was performed using a Minispec LF50 (Bruker biospin, BCA LF50) device to separately measure whole-body fat mass and lean mass (muscle and bone), and the change rates before and after two weeks of drug administration were determined.

As shown in FIG. 6d, compared with the control (vehicle-administered group), both 15 nmol/kg Tirzepatide mimetic and 60 nmol/kg MG12 treatment produced similar body weight reductions of about 23% over two weeks. Furthermore, as shown in FIG. 6e, the fat mass reduction rate was about 44% for Tirzepatide mimetic but about 52% for MG12, confirming greater fat-specific reduction. In addition, muscle-sparing effect was measured as shown in FIG. 6f, showing that the muscle reduction rate was about 14% in the Tirzepatide mimetic group but only about 7% in the MG12 group.

### 5-4: Analysis of Visceral Fat Reduction Efficacy

The present inventors then examined whether the fat reduction effect of the bispecific fusion protein according to one embodiment of the present invention was due to subcutaneous fat reduction or visceral fat reduction. Specifically, after completion of the experiment of Experimental Example 5-3, the experimental animals were sacrificed, subcutaneous and visceral fat were excised, and their weights measured. The values were then divided by body weight to calculate fat ratios for each fat tissue.

As shown in the left graph of FIG. 6g, both Tirzepatide mimetic and MG12 showed a tendency for decreased or similar subcutaneous fat ratios compared with the control (vehicle group), but the changes were not statistically significant. In contrast, as shown in the right graph of FIG. 6g, the visceral fat ratio was reduced to normal level of the control group, by Tirzepatide mimetic compared with the vehicle group. Importantly, in the group administered with the bispecific fusion protein (MG12) according to one embodiment of the present invention, visceral fat content was even lower than that in the Tirzepatide mimetic group, demonstrating that MG12 is highly effective in reducing visceral fat in obese animals.

### Experimental Example 6: Analysis of Therapeutic Effects on Endotoxemia and Liver Injury

If obesity is left untreated, it may progress to metabolic dysfunction-associated steatoic liver disease (MASLD, NAFLD), metabolic dysfunction-associated steatohepatitis (MASH, NASH), or even irreversible liver damage such as liver cirrhosis. Accordingly, the present inventors investigated whether administration of the bispecific fusion protein (MG12) according to one embodiment of the present invention could suppress obesity-associated symptoms, particularly increased metabolic endotoxemia and liver injury. To this end, Tirzepatide mimetic (a GLP-1/GIPR dual agonist of equivalent efficacy) and MG12 were administered for two weeks, after which serum was collected for component analysis. Endotoxemia was analyzed using a mouse LPS ELISA kit (CSB-E13066m, Cusabio), and liver injury was analyzed by measuring ALT levels using a dry-chem analyzer (DRI-CHEM NX-500, FUJI).

As shown in FIG. 7a, diet-induced obesity (DIO) increased serum LPS levels, inducing endotoxemia. Tirzepatide mimetic administration did not reduce this increase; however, administration of the bispecific fusion protein (MG12) reduced LPS levels to those of the control group. Additionally, as shown in FIG. 7b, diet-induced obesity caused liver injury, resulting in significantly increased ALT levels. Tirzepatide mimetic treatment reduced ALT levels to about half compared with the vehicle group, whereas administration of the bispecific fusion protein (MG12) according to one embodiment of the present invention significantly further reduced ALT levels compared with the Tirzepatide mimetic group.

### Experimental Example 7: Analysis of Glucose Uptake Effect

To verify whether the bispecific fusion protein according to one embodiment of the present invention can be used for the treatment of diabetes, the present inventors performed a 2-NBDG glucose uptake assay using mouse pre-adipocytes (3T3-L1 cells) and rat myoblasts (L6-GLUT4myc cells, provided by the laboratory of Amira Klip, PhD, Hospital for Sick Children).

Specifically, confluent 3T3-L1 pre-adipocytes cultured in 24-well plates were differentiated into adipocytes by replacing the culture medium with 24-differentiation induction medium [DMEM containing 10% FBS (Cytiva, USA), 1% penicillin/streptomycin (P/S, Cytiva, USA), 1 µM dexamethasone (DEXA, Sigma, USA), 0.5 mM 3-isobutyl-1-methylxanthine (IBMX, Sigma, USA), 2 µM Rosiglitazone (Cayman, Germany), and 1 µg/mL insulin (Sigma, USA)], and then culturing under 5% CO₂ conditions for 11 days. After differentiation, cells were starved of insulin and treated with 300 nM of the bispecific fusion protein MG12 according to one embodiment of the present invention and 10 µg/mL insulin, followed by incubation in glucose-free DMEM containing 100 µg/mL of 2-(N-(7-Nitrobenz-2-oxa-1,3-diazol-4-yl)amino)-2-deoxyglucose (2-NBDG, Cayman, USA). As positive controls, the same concentration (300 nM) of Semaglutide, and Dapiglutide were used. After 30 minutes of incubation, the cells were washed with glucose-free DMEM medium and incubated with lysis buffer (RIPA buffer) for 20 minutes. Fluorescence signals were measured at an excitation wavelength of 485 nm and an emission wavelength of 535 nm using a microplate reader (SpectraMax M3, USA).

Similarly, rat L6-GLUT4myc muscle cells were seeded into 24-well plates at a density of 2×10⁴ cells/well, and after two days the medium was replaced with α-MEM medium (Gibco, USA) containing 2% FBS (Cytiva, USA) and 1% penicillin/streptomycin, followed by differentiation into myotubes for 5 days. After differentiation, 300 nM of the bispecific fusion protein MG12 according to one embodiment of the present invention and 10 µg/mL insulin were added, followed by treatment with 2-NBDG at a concentration of 100 µg/mL for 30 minutes. After washing with α-MEM medium, cells were lysed and analyzed using a microplate reader (SpectraMax M3, USA) to measure intracellular 2-NBDG levels at 485 nm (excitation) and 535 nm (emission).

As shown in FIG. 8, the bispecific fusion protein (MG12) according to one embodiment of the present invention exhibited superior glucose uptake efficiency not only in differentiated adipocytes but also in myofibers, compared to GLP-1-Fc, GLP-2-Fc, conventional GLP-1 analogues (Semaglutide), and the GLP-1R/GLP-2R dual agonist (Dapiglutide).

### Experimental Example 8: Animal Experiment for Verification of Anti-Diabetic Therapeutic Effects

### 8-1: Analysis of Fasting Blood Glucose, Serum Insulin Levels, HOMA-IR, and HOMA-β Scores

To examine whether the bispecific fusion protein according to one embodiment of the present invention not only lowers blood glucose but also improves insulin resistance, the present inventors conducted animal experiments using diabetic model mice.

Specifically, 14-week-old male db/db mice (Janvier Labs, France) were subcutaneously injected every three days for 12 weeks with designated doses of Semaglutide, Tirzepatide, or the bispecific fusion protein MG12-8 according to one embodiment of the present invention.

Subsequently, non-fasting blood glucose levels were measured at 25-day intervals (on days 25, 50, and 75 of the experiment), and HbA1c levels were measured monthly up to three months. Additionally, at the end of the experiment, fasting blood glucose and serum insulin concentrations were determined, and HOMA-IR and HOMA-β scores were calculated.

As shown in FIG. 9, at a dosage of 30 nmol/kg, the bispecific fusion protein (MG12) according to one embodiment of the present invention demonstrated significantly superior non-fasting blood glucose-lowering ability and HbA1c reduction compared to 30 nmol/kg Semaglutide or 15 nmol/kg Tirzepatide. Furthermore, not only was fasting blood glucose markedly reduced, but fasting serum insulin levels were also significantly increased. Moreover, while both 30 nmol/kg Semaglutide and 15 nmol/kg Tirzepatide exceeded an insulin resistance index (HOMA-IR) of 40 and thus failed to eliminate insulin resistance, the bispecific fusion protein according to one embodiment of the present invention lowered HOMA-IR scores to control levels. In addition, the HOMA-β score, which reflects β-cell function, was significantly higher with the bispecific fusion protein than with 30 nmol/kg Semaglutide or 15 nmol/kg Tirzepatide, suggesting its potential applicability not only for type 2 diabetes but also for type 1 diabetes therapy.

### 8-2: Histological Analysis

Subsequently, after completion of the above experiment, the present inventors sacrificed the experimental animals and performed histological analysis of pancreatic tissues.

Specifically, after euthanizing the animals under general anesthesia at the end of the animal study, the pancreas was excised, fixed in 4% paraformaldehyde, embedded in paraffin, sectioned transversely into 5 µm slices using a microtome, and then subjected to staining either with insulin alone, double staining with insulin and glucagon, or double staining with insulin and Ki-67. All double staining was carried out using fluorescent-labeled antibodies, followed by imaging with a confocal laser microscope.

As shown in FIG. 10, the bispecific fusion protein according to one embodiment of the present invention, unlike conventional antidiabetic agents such as Semaglutide and Tirzepatide, significantly increased the islet area, markedly increased the area of β-cells within the islets, and even reduced the α-cell population, while substantially increasing the number of Ki-67-positive cells per 10,000 µm². Semaglutide, used as a positive control, showed a significant increase, but its effect was lower than that of MG12 at the same dosage, whereas Tirzepatide showed no improvement compared with the control group.

These results suggest that the bispecific fusion protein according to one embodiment of the present invention provides a much higher protective effect on pancreatic β-cells than conventional antidiabetic agents.

As described above, the bispecific fusion protein of the present invention demonstrated a safe and highly effective ability to reduce body weight and visceral fat content in obese animals, and was confirmed to be a highly effective agent for the treatment of metabolic diseases such as diabetes-including type 2 as well as type 1 diabetes-and obesity-related conditions such as liver damage caused by persistent obesity.

The present invention has been described with reference to the foregoing embodiments and experimental examples, which are provided merely for illustrative purposes. It will be understood by those skilled in the art that various modifications and equivalent embodiments are possible therefrom. Accordingly, the true technical scope of the present invention should be determined by the technical spirit set forth in the appended claims.

### INDUSTRIAL APPLICABILITY

The bispecific fusion protein according to one embodiment of the present invention can be used in medicine, in particular, as a therapeutic agent for metabolic diseases such as obesity, diabetes, and metabolic dysfunction-associated steatohepatitis (MASH).

## Claims

1. A bispecific fusion protein comprising a fusion of a GLP-1 analogue and a GLP-2 analogue, wherein the activity of the GLP-1 analogue is at least 40% relative to wild-type GLP-1 (human GLP-1 peptide), and the activity of the GLP-2 analogue is 50% or less relative to wild-type GLP-2 (human GLP-2 peptide).

2. A bispecific fusion protein produced by dimerization of a first fusion protein in which a GLP-1 analogue is linked to the antibody Fc region and a second fusion protein in which a GLP-2 analogue is linked to the antibody Fc region, wherein the activity of the GLP-1 analogue is at least 40% relative to wild-type GLP-1 (human GLP-1 peptide), and the activity of the GLP-2 analogue is 50% or less relative to wild-type GLP-2 (human GLP-2 peptide).

3. The bispecific fusion protein according to claim 1 or 2, wherein the activity of the GLP-1 analogue under *in vitro* conditions is at least 80% relative to wild-type GLP-1.

4. The bispecific fusion protein according to claim 1 or 2, wherein the activity of the GLP-2 analogue under *in vitro* conditions is at least 2% relative to wild-type GLP-2.

5. The bispecific fusion protein according to claim 1 or 2, wherein a half-life extension moiety is added.

6. The bispecific fusion protein according to claim 5, wherein the half-life extension moiety is inserted between the GLP-1 analogue and the GLP-2 analogue, or is added to the N-terminus or C-terminus of the entire fusion protein.

7. The bispecific fusion protein according to claim 5, wherein the half-life extension moiety is an antibody Fc region, PEG, XTEN, PAS (Pro-Ala-Ser), ELP (elastin-like peptide), glycine-rich HAP (homo-amino-acid polymer), GLP (gelatine-like protein), or serum albumin.

8. The bispecific fusion protein according to claim 1, wherein the bispecific fusion protein is not a proglucagon or its analogue in which GLP-1 and GLP-2 are linked to each other by an intervention peptide, but rather the GLP-1 analogue and the GLP-2 analogue are directly linked, or the two peptides are linked by a linker peptide other than the intervention peptide.

9. The bispecific fusion protein according to claim 1 or 2, wherein the GLP-1 analogue is selected from the group consisting of GLP-1, Exendin-3, Exendin-4, GLP-1/Exendin-4 hybrid peptide, GLP-1-XTEN, Exendin-4-XTEN, Lixisenatide, Albiglutide, Liraglutide, Taspoglutide, or a continuous repeat unit of one or more thereof.

10. The bispecific fusion protein according to claim 1 or 2, wherein the GLP-2 analogue is GLP-2, a GLP-2^{A2G} mutant, a GLP-2^{N16G_L17Q} mutant, a GLP-2^{A2G_N16G_L17Q} mutant, Glepaglutide, or GLP-2 analogue 10.

11. The bispecific fusion protein according to claim 2, wherein the antibody Fc region is a hybrid Fc region in which Fc regions of two or more isotypes are mixed.

12. The bispecific fusion protein according to claim 11, wherein the hybrid Fc region comprises the amino acid sequence selected from the group consisting of SEQ ID NOs: 12 to 16.

13. The bispecific fusion protein according to claim 2, wherein the antibody Fc region is an antibody Fc region having a heterodimerization moiety.

14. The bispecific fusion protein according to claim 13, wherein the heterodimerization moiety is Knobs-into-Holes (KiH), KiH_{S-S}, HA-TF, ZW1, 7.8.60, DD-KK, EW-RVT, EW-RVT_{S-S}, SEED, or A107.

15. A composition comprising the bispecific fusion protein according to any one of claims 1 to 14.

16. A pharmaceutical composition for treating a metabolic disease, comprising as an active ingredient the bispecific fusion protein according to any one of claims 1 to 14.

17. The pharmaceutical composition according to claim 16, wherein the metabolic disease is obesity, diabetes, metabolic syndrome, metabolic dysfunction-associated fatty liver disease, metabolic dysfunction-associated steatohepatitis, liver fibrosis, or liver cirrhosis.

18. The pharmaceutical composition according to claim 17, wherein the diabetes is type 1 diabetes or type 2 diabetes.

19. A use of the bispecific fusion protein according to any one of claims 1 to 14 in the treatment of a metabolic disease.

20. The bispecific fusion protein according to claim 19, wherein the metabolic disease is obesity, diabetes, metabolic syndrome, metabolic dysfunction-associated fatty liver disease, metabolic dysfunction-associated steatohepatitis, liver fibrosis, or liver cirrhosis.

21. The bispecific fusion protein according to claim 20, wherein the diabetes is type 1 diabetes or type 2 diabetes.

22. A use of the bispecific fusion protein according to any one of claims 1 to 14 in the manufacture of a medicament for the treatment of a metabolic disease.

23. The use according to claim 22, wherein the metabolic disease is obesity, diabetes, metabolic syndrome, metabolic dysfunction-associated fatty liver disease, metabolic dysfunction-associated steatohepatitis, liver fibrosis, or liver cirrhosis.

24. The use according to claim 23, wherein the diabetes is type 1 diabetes or type 2 diabetes.

25. A method for treating a metabolic disease in a subject, comprising administering to the subject a therapeutically effective amount of the bispecific fusion protein according to any one of claims 1 to 14.

26. The method according to claim 25, wherein the metabolic disease is obesity, diabetes, metabolic syndrome, metabolic dysfunction-associated fatty liver disease, metabolic dysfunction-associated steatohepatitis, liver fibrosis, or liver cirrhosis.

27. The method according to claim 26, wherein the diabetes is type 1 diabetes or type 2 diabetes.
